# EUROPÄISCHE PATENTSCHRIFT

(11) **EP 3 380 477 B1**
(45) Veröffentlichungstag und Bekanntmachung des Hinweises auf die Patenterteilung: **06.01.2021**
(21) Anmeldenummer: 16800925.6
(22) Anmeldetag: 22.11.2016
(51) Int. Cl.: C07D 493/06

(54) **VERFAHREN ZUR HERSTELLUNG VON VERBINDUNGEN MIT 16-OXABICYCLO[10.3.1]PENTADECENGERÜST UND DEREN FOLGEPRODUKTEN**
METHOD FOR THE PREPARATION OF COMPOUNDS WITH 16-OXABICYCLO [10.3.1] PENTADECEN SCAFFOLD AND THEIR SECONDARY PRODUCTS
PROCEDE DE PREPARATION DE COMPOSES A SQUELETTE DE 16-OXABICYCLOO[10.3.1]PENTADECEN ET LEURS PRODUITS SECONDAIRES

(30) Priorität: 23.11.2015 EP 15195836
(43) Veröffentlichungstag der Anmeldung: 03.10.2018
(73) Patentinhaber: BASF SE, 67056 Ludwigshafen (DE)
(72) Erfinder: WERNER, Albert, Bishop, TX 78343 (US); BRU ROIG, Miriam, 69126 Heidelberg (DE); TELES, Joaquim Henrique, 67165 Waldsee (DE); RUEDENAUER, Stefan, 69469 Weinheim (DE); MAURER, Stephan, 67435 Neustadt (DE); DANZ, Manuel, 68723 Plankstadt (DE)
(86) Internationale Anmeldenummer: PCT/EP2016/078385
(87) Internationale Veröffentlichungsnummer: WO 2017/089327

(56) Entgegenhaltungen:
- EP-A1- 0 400 509
- JP-A- 2002 335 991
- JP-A- 2012 046 444

## Beschreibung

### HINTERGRUND DER ERFINDUNG

Die vorliegende Erfindung betrifft ein Verfahren zur Herstellung von Verbindungen mit 16-Oxabicyclo[10.3.1]pentadecengerüst, speziell von 14-Methyl-16-oxabicyclo[10.3.1]-pentadecenen, und deren Folgeprodukten.

### STAND DER TECHNIK

Makrocyclische Ketone mit 14- bis 18-gliedrigen Ringen, wie z. B. Cyclopentadecanon (Exalton), 3-Methylcyclopentadecanon (Muscon) und 3-Methylcyclopentadecenon (Dehydromuscon oder Muscenon®), sind begehrte Riech- oder Aromastoffe, deren synthetische Herstellung Gegenstand umfangreicher Untersuchungen war und ist. Insbesondere das nicht in der Natur vorkommende Gemisch aus 3-Methyl-cyclopentadec-4-en-1-on und 3-Methyl-cyclopentadec-5-en-1-on ist aufgrund seiner olfaktorischen Eigenschaften von besonderem Interesse. Dieses Gemisch aus 3-Methyl-cyclopentadec-4-en-1-on und 3-Methyl-cyclopentadec-5-en-1-on wird im Folgenden "Dehydromuscon" genannt. Die folgende Formel (A), in der das Symbol einmal für eine Einfachbindung und einmal für eine Doppelbindung steht, zeigt Dehydromuscon ohne Berücksichtigung von Positions- und Doppelbindungsisomeren. Die Formeln (A') und (A") zeigen die beiden Doppelbindungsisomere ohne Berücksichtigung der cis-trans-Isomerie der Doppelbindungen.

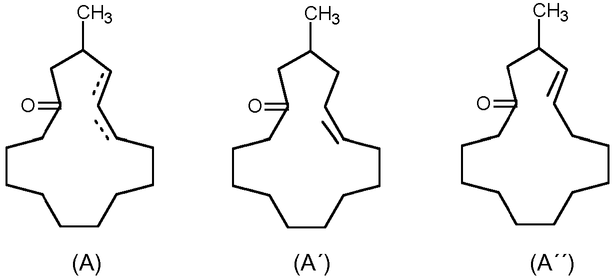

Die Darstellung (A) umfasst im Sinne der Erfindung die reine Verbindung (A'), die reine Verbindung (A") und beliebige Gemische aus (A') und (A"), wobei die Doppelbindungen jeweils sowohl eine cis- als auch eine trans-Geometrie haben können.

Die Synthese des Dehydromuscons ist u. a. in folgenden Dokumenten beschrieben: US 3,778,483; US 4,480,107 und CH 513791.

Die Isomere des 14-Methyl-16-oxabicyclo[10.3.1]pentadecens (D) sind wichtige Zwischenprodukte bei der Herstellung von Dehydromuscon (A) und von Muscon. So kann eine dreistufige Synthese von Dehydromuscon (A) ausgehen von 3-Methylcyclopentadecan-1,5-dion (B) und die folgenden Schritte umfassen: 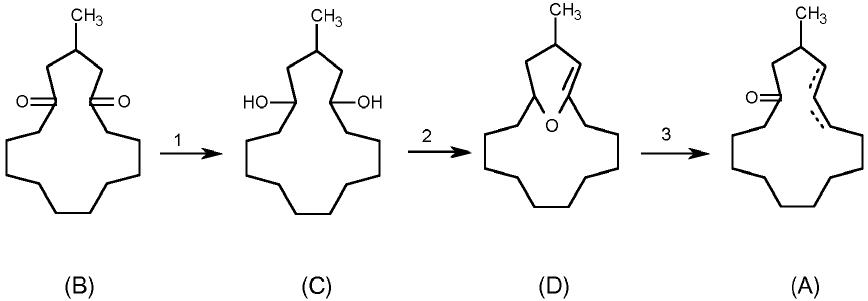
1. Reduktion des 3-Methylcyclopentadecan-1,5-dions (B) zum 3-Methyl-cyclopentadecan-1,5-diol (C).
2. Katalytische Dehydrierung und Dehydratisierung des 3-Methyl-cyclopentadecan-1,5-diols (C) zum 14-Methyl-16-oxabicyclo[10.3.1]pentadecen (D).
3. Umsetzung des 14-Methyl-16-oxabicyclo[10.3.1]pentadecens (D) zum Dehydromuscon (A).

In Stufe 2) können als Nebenprodukte das 3-Methylcyclopentadecan-1,5-dion (B) und 14-Methyl-16-Oxabicyclo[10.3.1]hexadecan (E) anfallen. Des Weiteren kann bei der Umsetzung des 3-Methyl-cyclopentadecan-1,5-diols (C) zum 14-Methyl-16-oxabicyclo[10.3.1]pentadecen (D) als Zwischenprodukt 3-Methyl-Cyclopentadecan-5-ol-1-on (F) gebildet werden:

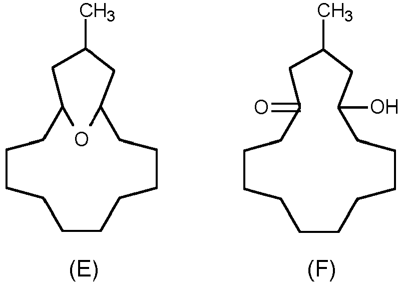

Die US 4,335,262 beschreibt unter anderem die Herstellung von Dehydromuscon (A) über Dehydrierung und Dehydratisierung des 3-Methyl-cyclopentadecan-1,5-diols (C) mit Raney-Kupfer in batch-Fahrweise zum 14-Methyl-16-oxabicyclo[10.3.1]pentadecen (D) (Beispiel 4 der US 4,335,262). Dabei wird 14-Methyl-16-oxabicyclo[10.3.1]penta-decen direkt durch Destillation aus dem Reaktionsgemisch abgetrennt. Anschließend kann 14-Methyl-16-oxabicyclo[10.3.1]pentadecen einer Umsetzung mit Phosphorsäure in Toluol unter Erhalt des Dehydromuscons (A) unterzogen werden (Beispiel 5 der US 4,335,262).

Nachteilig an diesem Verfahren ist jedoch die geringe Selektivität bezüglich des Dehydromuscons (A), da unter anderem nicht unerhebliche Mengen des gesättigten Ethers (E) erhalten werden, wodurch sich sowohl die Ausbeute als auch die Reinheit des Zielproduktes reduzieren. Weiterhin erschwert die hohe Viskosität der Reaktionsmischung eine technische Realisierung dieses Verfahrens.

EP 0400509 beschreibt ein Verfahren zur Herstellung von Muscon durch intramolekulare Aldolkondensation von offenkettigen 2,15-Diketonen. Die erhaltenen ungesättigten zyklischen Ketone werden anschließend hydriert.

Der vorliegenden Erfindung liegt die Aufgabe zugrunde, ein verbessertes Verfahren zur Herstellung von 16-Oxabicyclo[10.3.1]pentadecenen zur Verfügung zu stellen, bei denen das Ringkohlenstoffatom 14 unsubstituiert ist oder einen C₁-C₄-Alkylrest trägt. Die Synthese soll dabei von den entsprechenden Cyclopentadecan-1,5-diolen ausgehen. Der Erfindung liegt insbesondere die Aufgabe zugrunde, ein verbessertes Verfahren zur Herstellung von 14-Methyl-16-oxabicyclo[10.3.1]pentadec-12-en, ausgehend von 3-Methyl-cyclopentadecan-1,5-diol zur Verfügung zu stellen. Dabei sollen die 16-Oxabicyclo[10.3.1]pentadecene und speziell 14-Methyl-16-oxabicyclo[10.3.1]pentadecen mit hohem Umsatz in guter Ausbeute und Reinheit erzielt werden.

Es wurde nun gefunden, dass diese Aufgabe gelöst wird, wenn man die Umsetzung des entsprechenden Cyclopentadecan-1,5-diols zum unsubstituierten oder C₁-C₄-Alkyl-substituierten 16-Oxabicyclo[10.3.1]pentadecen an einem dehydrierenden und dehydratisierenden Katalysator und zusätzlich in Gegenwart eines hochsiedenden Lösungsmittels durchführt.

Speziell wurde gefunden, dass die unsubstituierten oder C₁-C₄-Alkyl-substituierten 16-Oxabicyclo[10.3.1]pentadecene, wie14-Methyl-16-oxabicyclo[10.3.1]pentadecen, bei zu langer Verweilzeit in der Reaktionszone in Kontakt mit dem Katalysator sowie dem während der Reaktion gebildeten Wasserstoff zu dem unerwünschten gesättigten Ether weiterreagiert. Durch den erfindungsgemäßen Zusatz eines hochsiedenden Lösungsmittels lässt sich erzielen, dass das Cyclopentadecan-1,5-diolausgangsmaterial, z. B. 3-Methyl-cyclopentadecan-1,5-diol, und der Katalysator in der Reaktionszone verbleiben, während die Verweilzeit des unsubstituierten oder C₁-C₄-Alkyl-substituierten 16-Oxabicyclo[10.3.1]pentadecens in der Reaktionszone minimiert werden kann. Auf diese Weise lassen sich zum einen hohe Ausbeuten bezogen auf das eingesetzte Cyclopentadecan-1,5-diol bei gleichzeitig verbesserter Ausbeute und Reinheit des unsubstituierten oder C₁-C₄-Alkyl-substituierten 16-Oxabicyclo[10.3.1]pentadecens erzielen. Durch den erfindungsgemäßen Zusatz eines hochsiedenden Lösungsmittels kann zudem die Durchmischung des Reaktionsgemischs in der Reaktionszone verbessert werden (bzw. die Viskosität des Reaktionsgemischs kann verringert werden).

Insbesondere wird zur Durchführung des erfindungsgemäßen Verfahrens eine Vorrichtung eingesetzt, die eine Reaktionszone und eine daran anschließende Destillationszone umfasst. Im Speziellen befindet sich nach Beginn der Umsetzung in der Reaktionszone ein Teil des unsubstituierten oder C₁-C₄-Alkyl-substituierten 16-Oxabicyclo-[10.3.1]pentadecens in der Destillationszone, auch dann, wenn dieser kein unsubstituiertes oder C₁-C₄-Alkyl-substituiertes 16-Oxabicyclo[10.3.1]pentadecen entnommen wird. Auch durch diese Vorgehensweise lässt sich die Verweilzeit des unsubstituierten oder C₁-C₄-Alkyl-substituierten 16-Oxabicyclo[10.3.1]pentadecens in der Reaktionszone und somit seine thermische Belastung minimieren.

Speziell dient das erfindungsgemäße Verfahren zur Herstellung von 14-Methyl-16-oxabicyclo[10.3.1]pentadec-12-en, ausgehend von 3-Methyl-cyclopentadecan-1,5-diol. In dieser speziellen Ausführung befindet sich nach Beginn der Umsetzung in der Reaktionszone ein Teil des 14-Methyl-16-oxabicyclo[10.3.1]pentadecens in der Destillationszone, auch dann, wenn dieser kein 14-Methyl-16-oxabicyclo[10.3.1]pentadecen entnommen wird.

### ZUSAMMENFASSUNG DER ERFINDUNG

Gegenstand der Erfindung ist ein Verfahren zur Herstellung von Verbindungen der allgemeinen Formel (I) 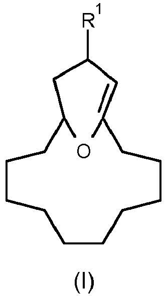 worin R¹ für Wasserstoff oder C₁-C₄-Alkyl steht,
und Folgeprodukten davon,
wobei man
a) ein Ausgangsmaterial bereitstellt, das eine Verbindung der allgemeinen Formel II enthält 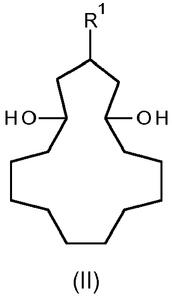
b) das in Schritt a) bereitgestellte Ausgangsmaterial in einer Reaktionszone einer Umsetzung bei einer Temperatur in einem Bereich von 100 bis 240 °C und einem Druck in einem Bereich von 0,1 bis 150 mbar in Gegenwart eines heterogenen Katalysators und eines Lösungsmittels oder eines Lösungsmittelgemisches unterzieht, das bei 180 °C einen Dampfdruck zwischen 10⁻⁵ und 100 mbar aufweist, und
c) aus dem Reaktionsgemisch die Verbindung der Formel (I) destillativ abtrennt. Die Erfindung betrifft weiterhin ein Verfahren, bei dem man zusätzlich
d) die Verbindungen der allgemeinen Formel (I) einer Umsetzung unter Erhalt wenigstens einer Verbindung der allgemeinen Formel (IV) unterzieht 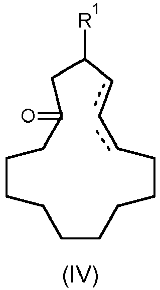 worin das Symbol ------ einmal für eine Einfachbindung und einmal für eine Doppelbindung steht und R¹ für Wasserstoff oder C₁-C₄-Alkyl steht.
   Die Erfindung betrifft weiterhin ein Verfahren, bei dem man zusätzlich
e) die Verbindungen der allgemeinen Formel (IV) einer Hydrierung unter Erhalt der Verbindung der allgemeinen Formel (V) unterzieht 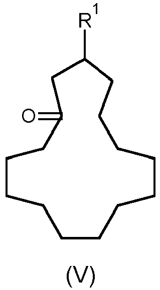 worin R¹ für Wasserstoff oder C₁-C₄-Alkyl steht.

### AUSFÜHRUNGSFORMEN DER ERFINDUNG

Das erfindungsgemäße Verfahren umfasst die folgenden Ausführungsformen.
1. Verfahren zur Herstellung von Verbindungen der allgemeinen Formel (I) 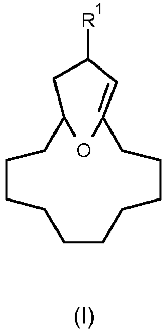 worin R¹ für Wasserstoff oder C₁-C₄-Alkyl steht,
   wobei man
   a) ein Ausgangsmaterial bereitstellt, das eine Verbindung der allgemeinen Formel II enthält 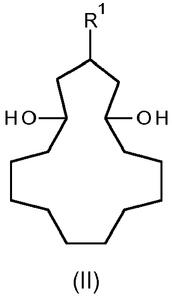
   b) das in Schritt a) bereitgestellte Ausgangsmaterial in einer Reaktionszone einer Umsetzung bei einer Temperatur in einem Bereich von 100 bis 240 °C und einem Druck in einem Bereich von 0,1 bis 150 mbar in Gegenwart eines heterogenen Katalysators und eines Lösungsmittels oder eines Lösungsmittelgemisches unterzieht, das bei 180 °C einen Dampfdruck im Bereich von 10⁻⁵ bis 100 mbar aufweist, und
   c) aus dem Reaktionsgemisch die Verbindung der Formel (I) destillativ abtrennt.
2. Verfahren nach Ausführungsform 1, wobei R¹ für Wasserstoff oder Methyl, insbesondere für Methyl, steht.
3. Verfahren nach Ausführungsform 1 oder 2, wobei die Umsetzung in Schritt b) eine erste Phase umfasst, während der noch keine die Verbindung der Formel (I) enthaltende Fraktion aus dem Reaktionsgemisch destillativ abgetrennt wird.
4. Verfahren nach einer der vorhergehenden Ausführungsformen, wobei die destillative Abtrennung einer die Verbindung der Formel (I) enthaltenden Fraktion aus der Reaktionszone im Schritt c) phasenweise oder kontinuierlich erfolgt.
5. Verfahren nach einer der Ausführungsformen 1 bis 4, wobei der Dampfdruck des in Schritt b) eingesetzten Lösungsmittels niedriger ist als der Dampfdruck des Diols (II).
6. Verfahren nach einer der Ausführungsformen 1 bis 4, wobei der Dampfdruck des in Schritt b) eingesetzten Lösungsmittels zwischen dem Dampfdruck der Verbindung (I) und dem Dampfdruck der Verbindung (II) liegt.
7. Verfahren nach einer der Ausführungsformen 1 bis 4, wobei der Dampfdruck des in Schritt b) eingesetzten Lösungsmittels zwischen dem Dampfdruck der Verbindung (I) und dem Dampfdruck der Verbindung (III) 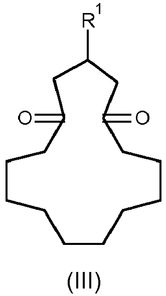 worin R¹ für Wasserstoff oder C₁-C₄-Alkyl steht,
   liegt.
8. Verfahren nach einer der vorhergehenden Ausführungsformen, wobei das in Schritt b) eingesetzte Lösungsmittel ausgewählt ist unter
   - aliphatischen, cycloaliphatischen und aromatischen Kohlenwasserstoffen,
   - aliphatischen, cycloaliphatischen und aromatischen ein- und mehrwertigen Alkoholen,
   - Etheralkoholen, Polyetherpolyolen und deren Mono- und Dialkylethern, aromatischen Ethern und offenkettigen aliphatischen Ethern,
   - Ketonen,
   - Estern,
   - Mischungen davon.
9. Verfahren nach einer der vorhergehenden Ausführungsformen, wobei das in Schritt b) eingesetzte Lösungsmittel ausgewählt ist unter
   - C₁₀-C₃₀-Alkanen,
   - C₆-C₃₀-Alkanolen,
   - C₂-C₃₀-Alkandiolen,
   - Polyalkylenglycolen und deren Mono- und Dialkylethern,
   - Mischungen davon.
10. Verfahren nach einer der Ausführungsformen 1 bis 9, wobei die Abtrennung in Schritt c) durch einstufige Destillation erfolgt.
11. Verfahren nach Ausführungsform 10 zur Herstellung von 14-Methyl-16-oxabicyclo[10.3.1]pentadecen (I.1), wobei in Schritt c) aus dem Reaktionsgemisch die Verbindung der Formel (I.1) durch einstufige Destillation abgetrennt wird und das abgetrennte Produkt die folgenden Verbindungen, jeweils bezogen auf das Gesamtgewicht des abgetrennten Produkts, enthält:
   14-Methyl-16-oxabicyclo[10.3.1]pentadecen (1.1): 75 - 95 Gew.-%,
   3-Methyl-cyclopentadecan-1,5-diol (II.1): 0 - 5 Gew.-%,
   3-Methylcyclopentadecan-1,5-dion (III.1): 1 - 10 Gew.-%,
   14-Methyl-16-Oxabicyclo[10.3.1]hexadecan (VI.1): 0 - 15 Gew.-%.
12. Verfahren nach einer der Ausführungsformen 1 bis 9, wobei die Abtrennung in Schritt c) eine fraktionierte Destillation umfasst.
13. Verfahren nach Ausführungsform 12, wobei in Schritt c) zur destillativen Abtrennung einer die Verbindung der Formel (I) enthaltenden Fraktion wenigstens eine Destillationskolonne eingesetzt wird, vorzugsweise eine Destillationskolonne, die wenigstens 10 theoretische Böden aufweist.
14. Verfahren nach Ausführungsform 12 oder 13, wobei bei der Destillation das Verhältnis von abgetrenntem Strom zu in die Kolonne zurückgeführtem Strom im Bereich von 1 : 1 bis 1 : 30, ganz speziell im Bereich von 1 : 1 bis 1 : 20, liegt.
15. Verfahren nach einer der Ausführungsformen 12 bis 14 zur Herstellung von 14-Methyl-16-oxabicyclo[10.3.1]pentadecen (I.1), wobei in Schritt c) aus dem Reaktionsgemisch die Verbindung der Formel (I) durch fraktionierte Destillation abgetrennt wird und das abgetrennte Produkt die folgenden Verbindungen, jeweils bezogen auf das Gesamtgewicht des abgetrennten Produkts, enthält:
   - 14-Methyl-16-oxabicyclo[10.3.1]pentadecen (1.1): 80 - 99 Gew.-%,
   - 3-Methyl-cyclopentadecan-1,5-diol (II.1): 0 - 5 Gew.-%,
   - 3-Methylcyclopentadecan-1,5-dion (III.1): 0 - 5 Gew.-%, bevorzugt 0 bis 1 Gew.-%,
   - 14-Methyl-16-Oxabicyclo[10.3.1]hexadecan (VI.1): 0 - 15 Gew.-%, bevorzugt 0 bis 10 Gew.-%,
   - Lösungsmittel: 0 - 5 Gew.-%, bevorzugt 0 bis 1 Gew.-%,
   - 3-Methyl-Cyclopentadecan-5-ol-1-on (VII.1): 0 - 5 Gew.-%.
16. Verfahren nach einer der vorhergehenden Ausführungsformen, wobei in Schritt b) der Gehalt des Reaktionsgemischs an dem Lösungsmittel immer bei mindestens 20 Gew.-%, bevorzugt mindestens 30 Gew.-%, insbesondere mindestens 50 Gew.-%, bezogen auf das Gesamtgewicht des Reaktionsgemischs in der Reaktionszone, gehalten wird.
17. Verfahren nach einer der vorhergehenden Ausführungsformen, wobei als Katalysator in Schritt b) ein kupferhaltiger Katalysator, vorzugsweise Raney-Kupfer, eingesetzt wird.
18. Verfahren zur Herstellung von Folgeprodukten der Verbindung der Formel (I) wie in einem der vorhergehenden Ausführungsformen definiert, wobei man zusätzlich
   d) die Verbindungen der allgemeinen Formel (I) einer Umsetzung unter Erhalt wenigstens einer Verbindung der allgemeinen Formel (IV) unterzieht 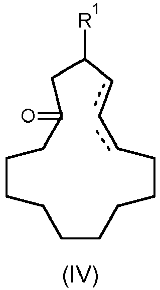 worin das Symbol ------ einmal für eine Einfachbindung und einmal für eine Doppelbindung steht und R¹ für Wasserstoff oder C₁-C₄-Alkyl steht.
19. Verfahren nach Ausführungsform 18, wobei man zusätzlich
   e) die Verbindungen der allgemeinen Formel (IV) einer Hydrierung unter Erhalt der Verbindung der allgemeinen Formel (V) unterzieht 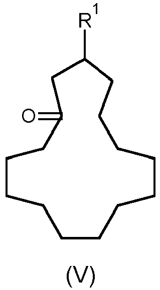 worin R¹ für Wasserstoff oder C₁-C₄-Alkyl steht.
20. Verfahren nach Ausführungsform 18 oder 19, wobei R¹ für Wasserstoff oder Methyl, insbesondere für Methyl, steht.

### BESCHREIBUNG DER ERFINDUNG

Das erfindungsgemäße Verfahren kann man prinzipiell als eine Reaktivdestillation verstehen, bei der jedoch Reaktionszone und anschließende Destillationszone nicht zwingen integral miteinander verbunden sind. Somit verbleibt der Katalysator sowie das als Edukt eingesetzte Cyclopentadecan-1,5-diol in der Reaktionszone (im Sumpf), während die Verweilzeit des Produktes in der Reaktionszone minimiert wird. Dies gelingt durch die Zugabe eines geeigneten Lösungsmittels. Auf diese Weise erhält man einen hohen Umsatz an Cyclopentadecan-1,5-diol (II) bei gleichzeitig verbesserter Ausbeute und Reinheit des Produktes der Formel (I).

Das erfindungsgemäße Verfahren weist die folgenden Vorteile auf:
- Das erfindungsgemäße Verfahren ermöglicht gleichzeitig eine ausreichende Kontaktzeit des Cyclopentadecan-1,5-diols (II) mit dem Katalysator und eine geringe Kontaktzeit des Produktes in der Reaktionszone.
- Die Bedingungen bei der destillativen Abtrennung der Verbindung (I) können speziell dann milder gestaltet werden, wenn der Dampfdruck des in Schritt b) eingesetzten Lösungsmittels zwischen dem Dampfdruck der Verbindung (I) und dem Dampfdruck der Verbindung (II) liegt. Bevorzugt liegt der Dampfdruck des in Schritt b) eingesetzten Lösungsmittels zwischen dem Dampfdruck der Verbindung (I) und dem Dampfdruck der Verbindung (III). Somit sind niedrigere Temperaturen und/oder ein geringeres Vakuum erforderlich. In der speziellen Ausführungsform der Erfindung zur Herstellung von 14-Methyl-16-oxabicyclo[10.3.1]-pentadecen (I.1) aus 3-Methyl-cyclopentadecan-1,5-diol (II.1) liegt der Dampfdruck des in Schritt b) eingesetzten Lösungsmittels vorzugsweise zwischen dem Dampfdruck der Verbindung (I.1) und dem Dampfdruck der Verbindung (II.1). Besonders bevorzugt liegt der Dampfdruck des in Schritt b) eingesetzten Lösungsmittels dann zwischen dem Dampfdruck der Verbindung (I.1) und dem Dampfdruck der Verbindung (III.1).
- Durch den Einsatz von Lösungsmitteln kann zudem die Durchmischung der Reaktionsmischung im Sumpf verbessert werden.
- Das erfindungsgemäße Verfahren ermöglicht den Einsatz einer geringen Katalysatormenge bezogen auf die Menge des in der Reaktionszone befindlichen Cyclopentadecan-1,5-diols. Dieser Vorteil ergibt sich bereits bei einer diskontinuierlichen Fahrweise ohne Ergänzung von umgesetztem Cyclopentadecan-1,5-diol im Verlauf der Umsetzung. Deutlich größer ist dieser Vorteil bei einer Fahrweise, bei der Reaktionszone im Verlauf der Umsetzung weiteres Cyclopentadecan-1,5-diol zugeführt wird. Dies gilt speziell für eine kontinuierliche Fahrweise.
- Mit dem erfindungsgemäßen Verfahren lassen sich somit die Verbindungen der allgemeinen Formel (I) und speziell 14-Methyl-16-oxabicyclo[10.3.1]pentadecen (I.1) mit hohem Umsatz in guter Ausbeute und Reinheit erzielen.

Im Rahmen der vorlegenden Erfindung steht R¹ für C₁-C₄-Alkyl und vorzugsweise für Methyl, Ethyl, n-Propyl, Isopropyl oder n-Butyl.

Bevorzugt steht in den Verbindungen der Formeln (I), (II), (III), (IV), (V), (VI) und (VII) R¹ für Wasserstoff oder Methyl, insbesondere für Methyl.

In einer bevorzugten Ausführung betrifft die Erfindung ein Verfahren zur Herstellung von Verbindungen der Formel (I.1)

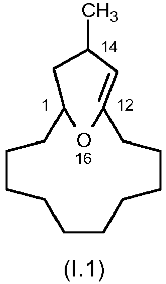

Die Verbindung der allgemeinen Formel (I.1) wird als 14-Methyl-16-oxabicyclo[10.3.1]-pentadec-12-en bezeichnet. Im Folgenden wird zum Teil auch auf die Angabe der Stellung der Doppelbindung verzichtet und synonym der Begriff 14-Methyl-16-oxabicyclo-[10.3.1]pentadecen verwendet.

Soweit im Folgenden nichts genauer angegeben ist, bezeichnen die allgemeinen Formeln (I) und (I.1) E/Z-Mischungen jedweder Zusammensetzung und die reinen Konformationsisomere. Weiterhin bezeichnen die allgemeinen Formeln (I) und (I.1) alle Stereoisomere in reiner Form sowie racemische und optisch aktive Mischungen der Verbindungen der Formeln (I) und (I.1).

Soweit im Folgenden nichts genauer angegeben ist, bezeichnet die allgemeine Formel (II) Gemische der möglichen cis/trans-Isomere jedweder Zusammensetzung sowie die reinen Konstitutionsisomere.

Bevorzugt enthält das in Schritt a) bereitgestellte Ausgangsmaterial eine Verbindung der allgemeinen Formel (II.1):

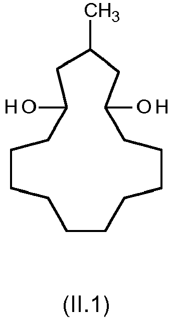

Die Verbindung der allgemeinen Formel (II.1) wird als 3-Methyl-cyclopentadecan-1,5-diol bezeichnet.

Insbesondere wird zur Durchführung des erfindungsgemäßen Verfahrens eine Vorrichtung eingesetzt, die eine Reaktionszone und eine daran anschließende Destillationszone umfasst. Im Speziellen befindet sich nach Beginn der Umsetzung in der Reaktionszone ein Teil der Verbindung der Formel (I) in der Destillationszone, auch dann, wenn dieser (noch) keine Verbindung der Formel (I) entnommen wird. Dies kann zu Beginn der Reaktion oder phasenweise während der destillativen Abtrennung der Verbindung der Formel (I) auftreten, z. B. wenn der Gehalt an der Verbindung (I) am Kolonnenkopf zu gering ist. Auch durch diese Vorgehensweise lässt sich die Verweilzeit der Verbindung der Formel (I) in der Reaktionszone und somit seine thermische Belastung minimieren.

Speziell dient das erfindungsgemäße Verfahren zur Herstellung von 14-Methyl-16-oxabicyclo[10.3.1]pentadec-12-en (I.1) ausgehend von 3-Methyl-cyclopentadecan-1,5-diol (II.1). In dieser speziellen Ausführung befindet sich nach Beginn der Umsetzung in der Reaktionszone ein Teil des 14-Methyl-16-oxabicyclo[10.3.1]pentadecens (I.1) in der Destillationszone, auch dann, wenn dieser kein 14-Methyl-16-oxabicyclo[10.3.1]-pentadecen (I.1) entnommen wird.

Das erfindungsgemäße Verfahren kann kontinuierlich, semikontinuierlich (semi-batch) oder diskontinuierlich (batch) durchgeführt werden.

Unter einer kontinuierlichen Fahrweise wird verstanden, dass abgesehen von einer Anfahrphase zu Beginn der Reaktion der Reaktionszone kontinuierlich die Verbindung der allgemeinen Formel (II) (speziell 3-Methyl-cyclopentadecan-1,5-diol (II.1)) zugeführt und aus dem Reaktionsgemisch kontinuierlich die Verbindung der allgemeinen Formel (I) (speziell 14-Methyl-16-oxabicyclo[10.3.1]pentadecen (1.1)) destillativ abgetrennt wird. Dabei erfolgt die Zuführung der Verbindung der allgemeinen Formel (II) vorzugsweise nach Maßgabe der abgetrennten Menge der Verbindung der allgemeinen Formel (I). Vorzugsweise befindet sich das Gemisch in der Reaktionszone dann im Wesentlichen im stationären Zustand, d. h. die Konzentration der Verbindung der allgemeinen Formel (II) und der Verbindung der allgemeinen Formel (I) im Reaktionsgemisch ist im Wesentlichen konstant.

Bei diskontinuierlicher Fahrweise wird der Reaktionszone ein Teil oder die Gesamtmenge der Verbindung der allgemeinen Formel (II) vor Beginn der Umsetzung zugeführt. Sobald sich die Verbindung der allgemeinen Formel (I) in ausreichender Menge gebildet hat, wird diese destillativ abgetrennt. Gegebenenfalls kann nach Abnahme des Gehalts an der Verbindung (II) in der Reaktionszone unter einen bestimmten Grenzwert frische Verbindung (II) in die Reaktionszone eingespeist werden. Dies kann sowohl einmalig als auch mehrmals erfolgen.

Es ist auch eine semikontinuierliche Fahrweise möglich, bei der einer der Schritte, Zugabe der Verbindung der allgemeinen Formel (II) oder die Abtrennung der Verbindung der allgemeinen Formel (I), kontinuierlich erfolgt und der andere diskontinuierlich.

### Schritt a):

Die Verbindungen der allgemeinen Formel (II) und ihre Herstellung sind prinzipiell bekannt. Dazu kann man eine Verbindung der allgemeinen Formel (III) 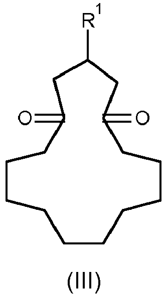 worin R¹ für Wasserstoff oder C₁-C₄-Alkyl steht, einer Umsetzung mit Wasserstoff in Gegenwart eines Hydrierkatalysators unterziehen.

Bevorzugt wird zur Bereitstellung von 3-Methyl-cyclopentadecan-1,5-diol (II.1) als Ausgangsmaterial in Schritt a) das 3-Methylcyclopentadecan-1,5-dion der Formel (III.1) 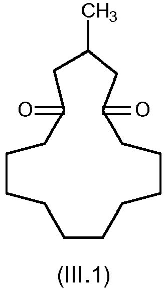 einer Umsetzung mit Wasserstoff in Gegenwart eines Hydrierkatalysators unterzogen.

Geeignete Hydrierkatalysatoren, die eine hohe Selektivität bezüglich der Hydrierung beider Ketogruppen zu Alkoholgruppen aufweisen, sind grundsätzlich die dem Fachmann für Hydrierreaktionen bekannten Übergangsmetallkatalysatoren. In der Regel umfasst der Katalysator wenigstens ein Übergangsmetall der Gruppen 7, 8, 9, 10 und 11 des Periodensystems nach IUPAC. Vorzugsweise weist der Katalysator wenigstens ein Übergangsmetall aus der Gruppe Mn, Re, Fe, Ru, Co, Rh, Ir, Ni, Pd, Pt, Cu und Au auf. Besonders bevorzugt weist der Katalysator Ni auf. Die Hydrierkatalysatoren bestehen aus den genannten Übergangsmetallen als solches oder umfassen die genannten Übergangsmetalle auf einen Träger aufgebracht, als Fällungskatalysatoren, als Raney-Katalysatoren oder als Mischungen davon.

Bevorzugt wird als Hydrierkatalysator ein Raney-Katalysator eingesetzt. Ein geeigneter Hydrierkatalysator ist Raney-Nickel.

Das Molverhältnis von Wasserstoff zu Verbindung (III) beträgt vorzugsweise 1000 : 1 bis 1 : 1, besonders bevorzugt 100 : 1 bis 5 : 1.

Die Hydrierung erfolgt bevorzugt bei einer Temperatur, die im Bereich von 10 bis 250 °C, besonders bevorzugt 20 bis 200 °C, liegt.

Bevorzugt erfolgt die Hydrierung in flüssiger Phase in Gegenwart eines Lösungsmittels.

Das zur Hydrierung eingesetzte Lösungsmittel ist vorzugsweise ausgewählt unter Wasser, aliphatischen C₁- bis C₅-Alkoholen, aliphatischen C₂- bis C₆-Diolen, Ethern und Gemischen daraus. Vorzugsweise ist das Lösungsmittel ausgewählt unter Methanol, Ethanol, n-Propanol, Isopropanol, n-Butanol, sek.-Butanol, Isobutanol und tert.-Butanol, Ethylenglykol, 1,3-Propandiol, 1,4-Butandiol, 1,5-Pentandiol, 1,6-Hexandiol, Tetrahydrofuran, 2-Methyltetrahydrofuran, Diethylether, Methyl-tert.-butylether und Gemischen daraus.

Das bei der Hydrierung der Verbindung der Formel (III) erhaltene Reaktionsgemisch kann vor seinem Einsatz als Ausgangsmaterial in Schritt a) des erfindungsgemäßen Verfahrens wenigstens einem Aufarbeitungsschritt unterzogen werden. Geeignete Aufarbeitungsschritte sind ausgewählt unter:
- Abtrennung des bei der Hydrierung eingesetzten Lösungsmittels,
- Abtrennung von nicht hydrierter Verbindung der Formel (III),
- Abtrennung von unerwünschten Nebenprodukten,
oder einer Kombination aus wenigstens zwei der zuvor genannten Maßnahmen.

Zu den unerwünschten Nebenprodukten bei der Hydrierung des 3-Methylcyclopentadecan-1,5-dions (III) zählen der teilhydrierte Ketonalkohol (3-Methyl-cyclopentadecan-5-ol-1-on) sowie ungesättigte Verbindungen.

Bevorzugt wird das Reaktionsgemisch aus der Hydrierung der Verbindung der allgemeinen Formel (III) einer destillativen Auftrennung unterzogen. Für diese Destillation kann im einfachsten Fall eine Destillationsvorrichtung zur einstufigen (einfachen) Destillation eingesetzt werden. Weitere geeignete Vorrichtungen zur destillativen Auftrennung des Reaktionsgemischs aus der Hydrierung der Verbindung (III) umfassen Destillationskolonnen, wie Bodenkolonnen, die mit Glocken, Siebplatten, Siebböden, Packungen, Füllkörpern, Ventilen, Seitenabzügen ausgerüstet sein können, Verdampfer, wie Dünnschichtverdampfer, Fallfilmverdampfer, Zwangsumlaufverdampfer, Sambay-Verdampfer und Kombinationen davon.

Die Destillationskolonnen können trennwirksame Einbauten aufweisen, die vorzugsweise ausgewählt sind unter Trennböden, geordnete Packungen, z. B. Blech- oder Gewebepackungen, wie Sulzer Mellapak®, Sulzer BX, Montz B1 oder Montz A3 oder Kühni Rombopak, oder regellose Schüttungen von Füllkörpern, wie z. B. Dixon-Ringen, Raschig-Ringen, High-Flow-Ringen oder Raschig-Super-Ringen.

Bevorzugt wird in Schritt a) des erfindungsgemäßen Verfahrens ein Material bereitgestellt, das die Verbindung der allgemeinen Formel II in einer Menge von wenigstens 50 Gew.-%, besonders bevorzugt wenigstens 70 Gew.-%, insbesondere wenigstens 90 Gew.-%, spezieller wenigstens 95 Gew.-%, bezogen auf das Gesamtgewicht des Ausgangsmaterials, aufweist. Dieses Material wird dann zur Umsetzung in Schritt (b) eingesetzt.

In einer bevorzugten Ausführung wird in Schritt a) des erfindungsgemäßen Verfahrens ein Material bereitgestellt, das 3-Methyl-cyclopentadecan-1,5-diol (II.1) in einer Menge von wenigstens 50 Gew.-%, besonders bevorzugt wenigstens 70 Gew.-%, insbesondere wenigstens 90 Gew.-%, spezieller wenigstens 95 Gew.-%, bezogen auf das Gesamtgewicht des Ausgangsmaterials, aufweist. Dieses Material wird dann vorzugsweise zur Umsetzung in Schritt (b) eingesetzt.

### Schritt b):

Erfindungsgemäß wird in Schritt b) das die Verbindung der Formel (II) enthaltende Ausgangsmaterial aus Schritt a) einer Umsetzung bei einer Temperatur in einem Bereich von 100 bis 240 °C und einem Druck in einem Bereich von 1 bis 150 mbar in Gegenwart eines Lösungsmittels oder eines Lösungsmittelgemisches unterzogen, das bei 180 °C einen Dampfdruck im Bereich von 10⁻⁵ bis 100 mbar aufweist.

Bei einer Temperatur im Bereich von 100 bis 240 °C und einem Druck im Bereich von 1 bis 150 mbar ergibt sich für die Umsetzung von 3-Methyl-cyclopentadecan-1,5-diol (II.1) zu 14-Methyl-16-oxabicyclo[10.3.1]pentadecen (I.1) folgende Siedereihenfolge (von Leicht- zu Schwersiedern): 14-Methyl-16-oxabicyclo[10.3.1]pentadecen (I.1), 3-Methylcyclopentadecan-1,5-dion (III.1), 3-Methyl-cyclopentadecan-1,5-diol (II.1).

Bei 180 °C weisen die Verbindungen folgende Dampfdrücke auf:
14-Methyl-16-oxabicyclo[10.3.1]pentadecen (I.1): 27 mbar
3-Methylcyclopentadecan-1,5-dion (III.1): 7 mbar
3-Methyl-cyclopentadecan-1,5-diol (II.1): 2 mbar

Grundsätzlich eignet sich für den Einsatz in dem erfindungsgemäßen Verfahren jedwedes Lösungsmittel, das einen Dampfdruck bei den Reaktionsbedingungen aufweist, der ausreichend unterhalb dem Dampfdruck der Verbindung der allgemeinen Formel (I), speziell des 14-Methyl-16-oxabicyclo[10.3.1]pentadecens (1.1), liegt. Falls das Lösungsmittel ein niedrigsiedendes Azeotrop mit der Verbindung der allgemeinen Formel (I), speziell dem 14-Methyl-16-oxabicyclo[10.3.1]pentadecen (I.1), bildet, kann die destillative Abtrennung der Verbindung (I) bzw. (I.1) zusammen mit dem Lösungsmittel oder Lösungsmittelgemisch erfolgen. Das Lösungsmittel sollte jedoch vorzugsweise kein hochsiedendes Azeotrop mit der Verbindung der allgemeinen Formel (I), speziell dem 14-Methyl-16-oxabicyclo[10.3.1]pentadecen (I.1), bilden, das bei 180 °C einen niedrigeren Dampfdruck als die Verbindung (II) bzw. (II.1) hat.

Die Siedepunkte der meisten Lösungsmittel sind Standardwerken, wie dem Handbook of Chemistry and Physics zu entnehmen, das von CRC Press, Inc., Boca Raton, Florida, USA in regelmäßigen Abständen aktualisiert herausgegeben wird. Dabei handelt es sich in der Regel um den Normalsiedepunkt bei 101.325 kPa. Der aktuelle Siedepunkt bei der Temperatur und dem Druck, die unter den Reaktionsbedingungen herrschen, kann unter Anwendung der Antoine-Gleichung oder der Claussius-Clapeyron-Gleichung ermittelt werden. Angaben dazu finden sich in Handbook of Chemistry and Physics, 76th Edition (1995 - 1996), 15-19, CRC Press, Inc., Boca Raton, Florida, USA. Die Ermittlung des aktuellen Siedepunkts eines bestimmten Lösungsmittels bei der Temperatur und dem Druck, die den Reaktionsbedingungen herrschen, gehört zudem zum Standardkönnen des Fachmanns.

In einer ersten Variante des erfindungsgemäßen Verfahrens wird in Schritt b) ein Lösungsmittel eingesetzt, das einen Dampfdruck aufweist, der niedriger ist als der Dampfdruck der Verbindung der allgemeinen Formel (II). Speziell dient das erfindungsgemäße Verfahren zur Herstellung von 14-Methyl-16-oxabicyclo[10.3.1]pentadecen (I.1) und wird in Schritt b) ein Lösungsmittel eingesetzt, das einen Dampfdruck aufweist, der niedriger ist als der Dampfdruck des 3-Methyl-cyclopentadecan-1,5-diols (II.1).

Der Dampfdruck bezieht sich dabei auf die Temperatur, die unter den Reaktionsbedingungen in Schritt b) herrscht.

In einer speziellen Ausgestaltung dieser ersten Variante wird die Verbindung der Formel (I.1) (14-Methyl-16-oxabicyclo[10.3.1]pentadecen) durch einstufige Destillation (d. h. ohne Rektifikation) aus dem Reaktionsgemisch abgetrennt.

In einer zweiten bevorzugten Variante des erfindungsgemäßen Verfahrens wird in Schritt b) ein Lösungsmittel eingesetzt, das einen Dampfdruck aufweist, der zwischen dem Dampfdruck der Verbindung (I) und der Verbindung (II) liegt. Speziell dient das erfindungsgemäße Verfahren zur Herstellung von 14-Methyl-16-oxabicyclo[10.3.1]-pentadecen (I.1), und es wird in Schritt b) ein Lösungsmittel eingesetzt, das einen Dampfdruck aufweist der zwischen dem Dampfdruck des 14-Methyl-16-oxabicyclo[10.3.1]pentadecens (I.1) und des 3-Methyl-cyclopentadecan-1,5-diols (II.1) liegt. Der Dampfdruck bezieht sich dabei auf die Temperatur, die unter den Reaktionsbedingungen in Schritt b) herrscht. In dieser Variante gelingt es in vorteilhafter Weise, dass bei der destillativen Abtrennung des 14-Methyl-16-oxabicyclo[10.3.1]pentadecens (I.1) in Schritt c) das nichtumgesetzte 3-Methyl-cyclopentadecan-1,5-diol (II.1), der Katalysator und das Lösungsmittel im Wesentlichen in der Reaktionszone verbleiben. Das dabei erhaltene Produktgemisch kann neben 14-Methyl-16-oxabicyclo[10.3.1]pentadecen (I.1) noch 14-Methyl-16-oxabicyclo[10.3.1]hexadecan (VI.1) und/oder 3-Methylcyclopentadecan-1,5-dion (III.1) enthalten.

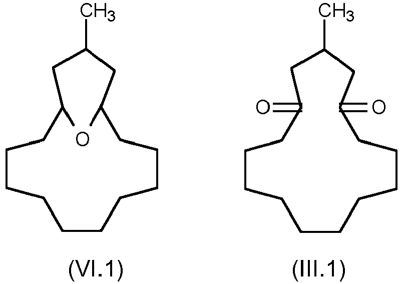

Gewünschtenfalls kann das Produktgemisch, das neben 14-Methyl-16-oxabicyclo-[10.3.1]pentadecen (I.1) noch 14-Methyl-16-oxabicyclo[10.3.1]hexadecan (VI.1) und/oder 3-Methylcyclopentadecan-1,5-dion (III.1) enthalten kann, einer destillativen Auftrennung unterzogen werden.

In einer dritten besonders bevorzugten Variante des erfindungsgemäßen Verfahrens wird in Schritt b) ein Lösungsmittel eingesetzt, das einen Dampfdruck aufweist, der zwischen dem Dampfdruck der Verbindung (I) und dem Dampfdruck der Verbindung (III) liegt. Speziell dient das erfindungsgemäße Verfahren zur Herstellung von 14-Methyl-16-oxabicyclo[10.3.1]pentadecen (I.1), und es wird in Schritt b) ein Lösungsmittel eingesetzt, das einen Dampfdruck aufweist, der zwischen dem Dampfdruck des 14-Methyl-16-oxabicyclo[10.3.1]pentadecens (I.1) und des 3-Methylcyclopentadecan-1,5-dions (III.1) liegt. Der Dampfdruck bezieht sich dabei auf die Temperatur, die unter den Reaktionsbedingungen in Schritt b) herrscht. In dieser Variante gelingt es in vorteilhafter Weise, dass bei der destillativen Abtrennung des 14-Methyl-16-oxabicyclo[10.3.1]pentadecens (I.1) in Schritt c) das nichtumgesetzte 3-Methyl-cyclopentadecan-1,5-diol (II.1), der Katalysator und das Lösungsmittel und, soweit vorhanden, das 3-Methylcyclopentadecan-1,5-dion (III.1) im Wesentlichen in der Reaktionszone verbleiben.

Erfindungsgemäß wird in Schritt b) ein Lösungsmittel oder ein Lösungsmittelgemisch eingesetzt, das bei 180 °C einen Dampfdruck im Bereich von 10⁻⁵ bis 100 mbar aufweist. Bevorzugt weist das Lösungsmittel oder Lösungsmittelgemisch bei 180 °C einen Dampfdruck im Bereich von 10⁻⁴ bis 100 mbar, insbesondere von 10⁻³ bis 100 mbar, auf.

Bevorzugt ist das in Schritt b) eingesetzte Lösungsmittel ausgewählt unter
- aliphatischen, cycloaliphatischen und aromatischen Kohlenwasserstoffen,
- aliphatischen, cycloaliphatischen und aromatischen ein- und mehrwertigen Alkoholen,
- Etheralkoholen, Polyetherpolyolen und deren Mono- und Dialkylethern, aromatischen Ethern und offenkettigen aliphatischen Ethern,
- Ketonen,
- Estern,
- Mischungen davon.

Besonders bevorzugt ist das in Schritt b) eingesetzte Lösungsmittel ausgewählt unter
- C₁₀-C₃₀-Alkanen,
- C₆-C₃₀-Alkanolen,
- C₂-C₃₀-Alkandiolen,
- Polyalkylenglycolen und deren Mono- und Dialkylethern,
- Mischungen davon.

Wenn das in Schritt b) eingesetzte Lösungsmittel wenigstens ein C₁₀-C₃₀-Alkan umfasst oder aus einem C₁₀-C₃₀-Alkan besteht, so ist dieses linear oder verzweigt und ist vorzugsweise ausgewählt unter C₁₂-C₂₈-Alkanen, besonders bevorzugt C₁₄-C₂₄-Alkanen.

Geeignete C₁₀-C₃₀-Alkane sind z. B. n-Decan, n-Undecan, n-Dodecan, n-Tridecan, n-Tetradecan, n-Pentadecan, n-Hexadecan, n-Heptadecan, n-Octadecan, n-Nonadecan, n-Eicosan, n-Heneicosan, n-Docosan, n-Tricosan, n-Tetracosan und deren Konstitutionsisomere.

Vorzugsweise wird als Lösungsmittel in Schritt b) wenigstens ein lineares C₁₄-C₂₄-Alkan eingesetzt.

Insbesondere ist das in Schritt b) eingesetzte Lösungsmittel ausgewählt unter n-Heptadecan, n-Octadecan, n-Nonadecan, n-Eicosan, n-Heneicosan und Mischungen davon.

Wenn das in Schritt b) eingesetzte Lösungsmittel wenigstens ein C₁₀-C₃₀-Alkanol umfasst oder aus einem C₁₀-C₃₀-Alkanol besteht, so sind die C₁₀-C₃₀-Alkylreste linear oder verzweigt und sind vorzugsweise ausgewählt unter C₁₂-C₂₈-Alkylresten, besonders bevorzugt C₁₄-C₂₄-Alkylresten.

Geeignete C₁₀-C₃₀-Alkylreste sind z. B. n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Heneicosyl, n-Docosyl, n-Tricosyl, n-Tetracosyl und deren Konstitutionsisomere.

Vorzugsweise wird als Lösungsmittel in Schritt b) wenigstens ein lineares C₁₄-C₂₄-Alkanol eingesetzt.

Besonders bevorzugt ist das in Schritt b) eingesetzte Lösungsmittel ausgewählt unter 1-Tetradecanol (Myristylalkohol), 1-Pentadecanol, 1-Hexadecanol (Cetylalkohol oder Palmitylalkohol), 1-Heptadecanol (Margarylalkohol), 1-Octadecanol (Stearylalkohol), Isostearylalkohol, 1-Eicosanol (Arachidylalkohol), 1-Docosanol (Behenylalkohol), 1-Tetracosanol (Lignocerylalkohol) und Mischungen davon.

Insbesondere ist das in Schritt b) eingesetzte Lösungsmittel ausgewählt unter 1-Pentadecanol, 1-Hexadecanol, 1-Heptadecanol, 1-Octadecanol und Mischungen davon.

Des Weiteren bevorzugt umfasst das in Schritt b) eingesetzte Lösungsmittel wenigstens ein Polyetherpolyol oder einen Monoalkylether oder einen Dialkylether davon.

Geeignete Polyetherpolyole und deren Mono- und Di-(C₁-C₆-alkylether) können linear oder verzweigt, vorzugsweise linear sein. Geeignete Polyetherole und deren Mono-und Di-(C₁-C₆-alkylether) weisen im Allgemeinen ein zahlenmittleres Molekulargewicht im Bereich von etwa 200 bis 2000, bevorzugt 280 bis 1000, auf. Bevorzugte Polyetherole sind Polyalkylenglycole, wie Polyethylenglycole, Polypropylenglycole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2-und 2,3-Butylenoxid. Geeignet sind Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Die Alkylenoxidcopolymere können die Alkylenoxideinheiten statistisch verteilt oder in Form von Blöcken einpolymerisiert enthalten. Besonders bevorzugt als Polyetherkomponente sind Ethylenoxid-Homopolymere.

Geeignet als Polyetherkomponente PE) sind weiterhin die Mono- und Di-(C₁-C₂-alkylether) der zuvor beschriebenen Polyetherole. Bevorzugt sind Polyalkylenglycolmonomethylether und Polyalkylenglycoldimethylether.

Geeignete Polyalkylenglycole sind die unter der Marke Lutrol E® von der BASF SE erhältlichen Polyethylenglycole. Besonders geeignet is Lutrol E® 400 mit im Mittel 8 Ethylenoxid-Wiederholungseinheiten.

Bevorzugt wird in Schritt b) der Gehalt des Reaktionsgemischs an dem Lösungsmittel immer bei mindestens 20 Gew.-%, bevorzugt mindestens 30 Gew.-%, insbesondere mindestens 50 Gew.-%, bezogen auf das Gesamtgewicht des Reaktionsgemischs in der Reaktionszone, gehalten.

Bevorzugt beträgt die Katalysatormenge in der Reaktionszone 0,001 bis 5 Gew.-%, besonders bevorzugt 0,01 bis 3 Gew.-%, bezogen auf das Gesamtgewicht des in der Reaktionszone befindlichen Reaktionsgemischs.

Bevorzugt beträgt die Katalysatormenge in der Reaktionszone 0,1 bis 15 Gew.-%, besonders bevorzugt 0,5 bis 10 Gew.-%, insbesondere 1 bis 5 Gew.-%, bezogen auf das Gewicht der maximal in der Reaktionszone befindlichen Verbindung der allgemeinen Formel (II), speziell 3-Methyl-cyclopentadecan-1,5-diol (II.1).

Die zuvor genannten Werte für die Katalysatormenge in der Reaktionszone gelten prinzipiell für die diskontinuierliche, semikontinuierliche und kontinuierliche Fahrweise. Die erfindungsgemäß eingesetzten Katalysatoren zeichnen sich durch gute Standzeiten aus, so dass über eine lange Reaktionsdauer frisches Cyclopentadecan-1,5-diol (II) in die Reaktionszone eingespeist werden kann, ohne dass die Katalysatoraktivität merklich abnimmt. Das erfindungsgemäße Verfahren ermöglicht somit die Herstellung von Verbindungen der allgemeinen Formel (I) aus Cyclopentadecan-1,5-diolen (II) mit sehr geringen Katalysatormengen bezogen auf den Gesamtumsatz.

Die als Reaktionszone in Schritt b) einsetzbaren Reaktoren unterliegen keinen besonderen Beschränkungen. Demgemäß können als Reaktoren mindestens ein Rührreaktor, mindestens ein Rohrreaktor oder mindestens ein Schlaufenreaktor eingesetzt werden. Die Reaktoren können mit mindestens einem internen und/oder mindestens einem externen Wärmetauscher ausgestattet sein. Ebenso ist es möglich, mindestens einen dieser Reaktoren so auszugestalten, dass er mindestens zwei unterschiedliche Zonen aufweist. Solche Zonen können sich in Reaktionsbedingungen, wie der Temperatur oder dem Druck und/oder in der Geometrie der Zone, wie dem Volumen oder dem Querschnitt, unterscheiden. Wird die Umsetzung in zwei oder mehr Reaktoren durchgeführt, können zwei oder mehr gleiche Reaktortypen oder mindestens zwei verschiedene Reaktortypen eingesetzt werden.

Das Cyclopentadecan-1,5-diol (II) wird zweckmäßigerweise in flüssiger Form in die Reaktionszone eingetragen. In einer ersten bevorzugten Ausführungsform wird eine Schmelze des Cyclopentadecan-1,5-diols (II) in die Reaktionszone eingetragen. In einer zweiten bevorzugten Ausführungsform wird das Cyclopentadecan-1,5-diol (II) als Lösung in dem eingesetzten Lösungsmittel in die Reaktionszone eingetragen. Wird das erfindungsgemäße Verfahren diskontinuierlich durchgeführt, so wird das Cyclopentadecan-1,5-diol (II) vorzugsweise teilweise oder vollständig in der Reaktionszone vorgelegt.

Wie ausgeführt, dient das erfindungsgemäße Verfahren speziell zur Herstellung von 14-Methyl-16-oxabicyclo[10.3.1]pentadecen (I.1) aus 3-Methyl-cyclopentadecan-1,5-diol (II.1). Wird das 3-Methyl-cyclopentadecan-1,5-diol (II.1) im Verlauf der Umsetzung in Schritt b) der Reaktionszone zugeführt, so wird in einer ersten bevorzugten Ausführungsform eine Schmelze des 3-Methyl-cyclopentadecan-1,5-diols (II.1) in die Reaktionszone eingetragen. Diese Variante ist geeignet bei diskontinuierlicher, semikontinuierlicher oder kontinuierlicher Fahrweise. Da in dieser Variante der Reaktionszone nicht zusätzlich das erfindungsgemäß eingesetzte hochsiedende Lösungsmittel zugeführt wird, kann dessen Aufpegelung wirksam vermieden werden.

In einer zweiten bevorzugten Ausführungsform wird das 3-Methyl-cyclopentadecan-1,5-diol (II.1) im Verlauf der Umsetzung in Schritt b) der Reaktionszone als Lösung in einem niedrigsiedenden Lösungsmittel zugeführt. Hierzu können dieselben niedrigsiedenden Lösungsmittel eingesetzt werden, wie zur Zuführung des Suspensionskatalysators in die Reaktionszone. Diese niedrigsiedenden Lösungsmittel können in der ersten Phase zu Beginn der Umsetzung destillativ entfernt werden. Weiterhin können diese niedrigsiedenden Lösungsmittel zusammen mit dem 14-Methyl-16-oxabicyclo[10.3.1]-pentadecen (I.1) am Kopf der Kolonne kondensiert und ausgetragen werden. Alternativ können die niedrigsiedenden Lösungsmittel auch gasförmig ausgetragen werden.

In einer dritten Ausführungsform wird das 3-Methyl-cyclopentadecan-1,5-diol (II.1) im Verlauf der Umsetzung in Schritt b) der Reaktionszone als Lösung in dem erfindungsgemäß eingesetzten (hochsiedenden) Lösungsmittel zugeführt. Diese Variante ist nicht bevorzugt für die kontinuierliche Zuführung des 3-Methyl-cyclopentadecan-1,5-diols (II.1), weil es zu einer Aufpegelung des Lösungsmittels in der Reaktionszone kommen kann. Möglich ist eine destillative Abtrennung dieses hochsiedenden Lösungsmittels gemeinsam mit dem 14-Methyl-16-oxabicyclo[10.3.1]pentadecen (I.1) in einer einstufigen Destillation.

Für die Umsetzung in Schritt b) wird ein heterogener Katalysator eingesetzt, der zur Dehydrierung und Dehydratisierung des Cyclopentadecan-1,5-diols (II) befähigt ist.

Bevorzugt enthalten die in Schritt b) eingesetzten Katalysatoren wenigstens ein Übergangsmetall der Gruppen 7, 8, 9, 10 und 11 des Periodensystems nach IUPAC. Weiter bevorzugt enthalten die in Schritt b) eingesetzten Katalysatoren mindestens ein Element, ausgewählt aus der Gruppe bestehend aus Cu, Co, Rh, Ir, Ni, Pd, Pt, Re, Fe, Ru und Au. Besonders bevorzugt enthalten die eingesetzten Katalysatoren Cu. In einer speziellen Ausführungsform ist Kupfer das einzig in der aktiven Masse des Katalysators eingesetzte Metall.

Die in Schritt b) eingesetzten Katalysatoren umfassen die genannten Übergangsmetalle, insbesondere die als bevorzugt genannten Übergangsmetalle, in der Regel als solche, auf einen Träger aufgebracht, als Fällkatalysatoren, als Raney-Katalysatoren oder als Mischungen davon. Speziell wird in Schritt b) Raney-Kupfer eingesetzt.

Als inertes Trägermaterial für die in Schritt b) eingesetzten Katalysatoren können praktisch alle Trägermaterialien des Standes der Technik, wie sie vorteilhaft bei der Herstellung von geträgerten Katalysatoren Verwendung finden, beispielsweise Kohlenstoff, SiO₂ (Quarz), Porzellan, Magnesiumoxid, Zinndioxid, Siliciumcarbid, TiO₂ (Rutil, Anatas), Al₂O₃ (Tonerde), Aluminiumsilikat, Steatit (Magnesiumsilikat), Zirkoniumsilikat, Cersilikat oder Mischungen dieser Trägermaterialien, eingesetzt werden. Bevorzugte Trägermaterialien sind Kohlenstoff, Aluminiumoxid und Siliciumdioxid.

Werden zwei oder mehr Metalle eingesetzt, so können diese separat oder als Legierung vorliegen. Hierbei ist es möglich, mindestens ein Metall als solches und mindestens ein anderes Metall als Raney-Katalysator oder mindestens ein Metall als solches und mindestens ein anderes Metall, aufgebracht auf mindestens einen Träger, oder mindestens ein Metall als Raney-Katalysator und mindestens ein anderes Metall, aufgebracht auf mindestens einen Träger, oder mindestens ein Metall als solches und mindestens ein anderes Metall als Raney-Katalysator und mindestens ein anderes Metall, aufgebracht auf mindestens einen Träger, einzusetzen.

Die Katalysatoren können als Formkörper eingesetzt werden, z. B. in Form von Kugeln, Ringen, Zylindern, Würfeln, Quadern oder anderen geometrischen Körpern. Ungeträgerte Katalysatoren können nach üblichen Verfahren geformt werden, z. B. durch Extrudieren, Tablettieren. Die Form geträgerter Katalysatoren wird durch die Form des Trägers bestimmt. Alternativ dazu kann der Träger vor oder nach dem Aufbringen der katalytisch aktiven Komponente(n) einem Formungsverfahren unterzogen werden. Die Katalysatoren können z. B. in Form von gepressten Zylindern, Tabletten, Pastillen, Wagenrädern, Ringen, Sternen oder Strangpresslingen, wie Vollsträngen, polylobären Strängen, Hohlsträngen und Wabenkörpern oder anderen geometrischen Körpern, eingesetzt werden.

Geeignete Reaktoren für die Umsetzung in Schritt b) sind dem Fachmann bekannte Reaktoren, die für Umsetzungen unter gleichzeitiger Verdampfung einer Komponente und/oder Freisetzung einer gasförmigen Komponente und/oder das Umsetzen unter vermindertem Druck geeignet sind. Dazu zählen z. B. Rührkessel (die auch als Rührkesselkaskaden ausgestaltet sein können), Rohrreaktoren, Rohrbündelreaktoren, Umlaufreaktoren.

Zur Zufuhr der für die Umsetzung in Schritt b) und die destillative Abtrennung der Verbindung (I), speziell des 14-Methyl-16-oxabicyclo[10.3.1]pentadecens (I.1), in Schritt c) erforderlichen Wärme kann einer oder können mehrere der Reaktoren mit wenigstens einer Heizvorrichtung versehen sein. Wird ein einzelner Reaktor eingesetzt, so ist dieser in der Regel mit einer Heizvorrichtung versehen. Werden mehrere Reaktoren eingesetzt, so ist in der Regel wenigstens der erste Reaktor, speziell alle Reaktoren, mit einer Heizvorrichtung versehen. Die Zufuhr der Wärme kann auch wenigstens teilweise durch Heizen eines externen Umlaufstroms oder durch interne Heizung in wenigstens einem der Reaktoren erfolgen. Zur internen Heizung können die dafür üblichen Vorrichtungen, im Allgemeinen Hohlkörpermodule, wie Field-Rohre, Rohrschlangen, Wärmetauscherplatten, eingesetzt werden. Alternativ kann die Umsetzung auch in einem geheizten Rohrbündelreaktor erfolgen.

Schritt b) des erfindungsgemäßen Verfahrens unter Verwendung wenigstens eines heterogenen Katalysators kann in Festbett- oder Suspensionsfahrweise durchgeführt werden. Die Festbettfahrweise kann dabei z. B. in Sumpf- oder in Rieselfahrweise durchgeführt werden.

Wird Schritt b) mit mindestens einem Suspensionskatalysator durchgeführt, so umfasst die Reaktionszone bevorzugt mindestens einen Rührreaktor. Die heterogenen Katalysatoren werden dabei zumeist in feinverteiltem Zustand eingesetzt und liegen im Reaktionsmedium feinteilig suspendiert vor. Dazu wird der Katalysator vorzugsweise als Suspension in dem erfindungsgemäß verwendeten Lösungsmittel oder einem davon verschiedenen niedrigsiedenden Lösungsmittel in die Reaktionszone eingespeist. Geeignete von den erfindungsgemäß verwendeten Lösungsmitteln verschiedene niedrigsiedende Lösungsmittel sind vorzugsweise ausgewählt unter Wasser, C₁- bis C₄-Alkanolen und Gemischen davon. Bevorzugt sind Wasser, Methanol und Mischungen davon. Diese niedrigsiedenden Lösungsmittel können vor bzw. zu Beginn der Umsetzung destillativ entfernt werden. In der Regel geht man so vor, dass man den Suspensionskatalysator in der Reaktionszone vorlegt. Diese Vorgehensweise ist unabhängig davon, dass man das erfindungsgemäße Verfahren diskontinuierlich, semikontinuierlich oder kontinuierlich durchführt. Die eingesetzten Katalysatoren zeichnen sich durch eine lange Standzeit aus. Dennoch ist es möglich, bei Abnahme der Katalysatoraktivität, speziell bei kontinuierlicher Fahrweise, frische Katalysatorsuspension in die Reaktionszone einzuspeisen. In diesem Falle setzt man den frisch eingespeisten Suspensionskatalysator vorzugsweise als Suspension in dem erfindungsgemäß eingesetzten Lösungsmittel ein.

### Schritt c):

In Schritt c) des erfindungsgemäßen Verfahrens wird aus dem in der Reaktionszone befindlichen Reaktionsgemisch eine die Verbindung der Formel (I) enthaltende Fraktion destillativ abgetrennt.

Speziell dient das erfindungsgemäße Verfahren zur Herstellung von 14-Methyl-16-oxabicyclo[10.3.1]pentadecen (I.1) aus 3-Methyl-cyclopentadecan-1,5-diol (II.1). Dann wird in Schritt c) des erfindungsgemäßen Verfahrens aus dem in der Reaktionszone befindlichen Reaktionsgemisch eine die Verbindung der Formel (I.1) (14-Methyl-16-oxabicyclo[10.3.1]pentadecen) enthaltende Fraktion destillativ abgetrennt.

Insbesondere wird zur Durchführung des erfindungsgemäßen Verfahrens eine Vorrichtung eingesetzt, die eine Reaktionszone und eine daran anschließende Destillationszone umfasst. In einer bevorzugten Ausführungsform umfasst die erfindungsgemäß zur Durchführung der Schritte b) und c) eingesetzte Vorrichtung einen Rührkessel oder eine Rührkesselkaskade, wobei der Rührkessel oder bei einer Rührkesselkaskade der letzte Rührkessel in Stromrichtung mit einer Destillationsvorrichtung versehen ist.

Die Umsetzung in Schritt b) sowie die destillative Abtrennung einer die Verbindung der Formel (I) (speziell 14-Methyl-16-oxabicyclo[10.3.1]pentadecen (1.1)) enthaltenden Fraktion in Schritt c) erfolgen vorzugsweise bei einer Temperatur in einem Bereich von 100 bis 240 °C. Besonders bevorzugt liegt die Temperatur in einem Bereich von 120 bis 220 °C.

Die Umsetzung in Schritt b) sowie die destillative Abtrennung einer die Verbindung der Formel (I) (speziell 14-Methyl-16-oxabicyclo[10.3.1]pentadecen (1.1)) enthaltenden Fraktion in Schritt c) erfolgen vorzugsweise bei einem Druck in einem Bereich von 0,1 bis 100 mbar. Besonders bevorzugt liegt der Druck in einem Bereich von 0,5 bis 50 mbar.

Bevorzugt erfolgt die erste Befüllung der Reaktionszone vor Beginn der Umsetzung bei einer Temperatur im Bereich von 10 bis 100 °C, bevorzugt 15 bis 70 °C. Die Temperatur richtet sich dabei nach den eingesetzten Komponenten des Reaktionsgemischs. Wird z. B. Hexadecanol als Lösungsmittel eingesetzt, so erfolgt die Befüllung zweckmäßigerweise oberhalb der Schmelztemperatur von 49 °C. Wird z. B. Methanol als Hilfslösungsmittel zur Einfüllung der Katalysatorsuspension eingesetzt, so erfolgt die Befüllung zweckmäßigerweise unterhalb der Siedetemperatur von ca. 67 °C. Die Temperatur kann während der Umsetzung in Schritt b) und der destillativen Abtrennung in Schritt c) auch einmal oder mehrmals schrittweise oder kontinuierlich erhöht werden, z. B. um die Abtrennung der während der Umsetzung gebildeten Verbindung der allgemeinen Formel (I) zu beschleunigen. Des Weiteren kann die Temperatur während der Umsetzung in Schritt b) und der destillativen Abtrennung in Schritt c) auch einmal oder mehrmals schrittweise oder kontinuierlich verringert werden, z. B. um der Reaktionszone Komponenten, wie frisches Cyclopentadecan-1,5-diol (II), frisches Lösungsmittel oder frischen Katalysator, zuzuführen und/oder um die Abtrennung der Verbindung der allgemeinen Formel (I) zu unterbrechen, z. B. um den Gehalt der Verbindung der allgemeinen Formel (I) in der Reaktionszone nach einer Phase der Abtrennung wieder zu erhöhen.

Bevorzugt erfolgt die erste Befüllung der Reaktionszone vor Beginn der Umsetzung bei Umgebungsdruck. Der Druck kann während Umsetzung in Schritt b) und der destillativen Abtrennung in Schritt c) auch einmal oder mehrmals schrittweise oder kontinuierlich reduziert werden, z. B. um die Abtrennung der während der Umsetzung gebildeten Verbindung der allgemeinen Formel (I) zu beschleunigen. Des Weiteren kann der Druck während der Umsetzung in Schritt b) und der destillativen Abtrennung in Schritt c) auch einmal oder mehrmals schrittweise oder kontinuierlich erhöht werden, z. B. um der Reaktionszone Komponenten, wie frisches Cyclopentadecan-1,5-diol (II), frisches Lösungsmittel oder frischen Katalysator, zuzuführen und/oder um die Abtrennung der Verbindung der allgemeinen Formel (I) zu unterbrechen, z. B. um den Gehalt der Verbindung der allgemeinen Formel (I) in der Reaktionszone nach einer Phase der Abtrennung wieder zu erhöhen.

Gegebenenfalls wird vor Beginn der eigentlichen Umsetzung zunächst der Druck in der Reaktionszone und der Destillationszone verringert (optionale Phase 1). Zusätzlich kann vor Beginn der eigentlichen Umsetzung die Temperatur in der Reaktionszone bereits erhöht werden. Diese Phase 1 ist jedoch dadurch gekennzeichnet, dass die Temperatur unterhalb von 100 °C und/oder dass der Druck oberhalb von 100 mbar liegt. In einer bevorzugten Ausführungsform wird in der Phase vor Beginn der Umsetzung zunächst der Druck reduziert, vorzugsweise auf einen Wert im Bereich von 100 bis 500 mbar, besonders bevorzugt 180 bis 300 mbar. Bevorzugt wird in dieser Phase die Temperatur gegenüber der Temperatur bei der ersten Befüllung der Reaktionszone nicht oder höchstens um 50 °C erhöht. In einer speziellen Ausführung wird die Umsetzung in Schritt b) mit mindestens einem Suspensionskatalysator durchgeführt, wobei der Katalysator in einem niedrigsiedenden Lösungsmittel in die Reaktionszone eingespeist wird. Bevorzugt werden diese niedrigsiedenden Lösungsmittel in der ersten Phase zu Beginn der Umsetzung destillativ entfernt.

Bevorzugt wird nach der Phase 1 der Druck in der Reaktionszone und der Destillationszone stufenweise oder kontinuierlich reduziert. Gleichzeitig oder unabhängig davon kann die Temperatur stufenweise oder kontinuierlich erhöht werden.

Gegebenenfalls umfasst die Umsetzung in Schritt b) eine Phase, während der die Temperatur in der Reaktionszone in einem Bereich von 100 bis 240 °C und der Druck in der Reaktionszone und der Destillationszone in einem Bereich von 0,1 bis 150 mbar liegt, in der aber noch keine die Verbindung der Formel (I) enthaltende Fraktion aus dem Reaktionsgemisch destillativ abgetrennt wird (optionale Phase 2). In dieser Phase wird in der Reaktionszone die Verbindung der allgemeinen Formel (I) (speziell 14-Methyl-16-oxabicyclo[10.3.1]pentadecen (I.1)) aus der Verbindung der allgemeinen Formel (II) (speziell 3-Methyl-cyclopentadecan-1,5-diol (11.1)) gebildet, so dass die Konzentration des Reaktionsgemischs an der Verbindung der allgemeinen Formel (I) (speziell 14-Methyl-16-oxabicyclo[10.3.1]pentadecen (1.1)) zunimmt. Im Speziellen befindet sich in der Phase 2 ein Teil der Verbindung der allgemeinen Formel (I) (speziell des 14-Methyl-16-oxabicyclo[10.3.1]pentadecens (I.1)) in der Destillationszone, auch dann, wenn dieser keine Verbindung der allgemeinen Formel (I) (speziell 14-Methyl-16-oxabicyclo[10.3.1]pentadecen (I.1)) entnommen wird. Durch diese Vorgehensweise lässt sich die Verweilzeit der Verbindung der allgemeinen Formel (I) (speziell des 14-Methyl-16-oxabicyclo[10.3.1]pentadecens (I.1)) in der Reaktionszone und somit seine thermische Belastung minimieren.

Die Umsetzung in Schritt b) umfasst eine Phase, während der die Temperatur in der Reaktionszone in einem Bereich von 100 bis 240 °C und der Druck in der Reaktionszone und der Destillationszone in einem Bereich von 0,1 bis 150 mbar liegen und in der die Verbindung der Formel (I) (speziell 14-Methyl-16-oxabicyclo[10.3.1]pentadecen (I.1)) destillativ aus dem Reaktionsgemisch abgetrennt wird (Phase 3).

Im einfachsten Fall der diskontinuierlichen Durchführung des erfindungsgemäßen Verfahrens ohne Ergänzung von umgesetztem Cyclopentadecan-1,5-diol (II) wird die Phase 3 einmal durchlaufen, bis das Cyclopentadecan-1,5-diol (II) in der Reaktionszone soweit wie möglich zur Verbindung (I) umgesetzt und dieser soweit wie möglich destillativ aus dem Reaktionsgemisch abgetrennt ist.

Im idealen Fall der kontinuierlichen Durchführung des erfindungsgemäßen Verfahrens wird die Phase 3 durchlaufen, bis trotz Zuführung von Cyclopentadecan-1,5-diol (II) und gegebenenfalls weiterer Komponenten (wie Lösungsmittel oder Katalysator) in die Reaktionszone die Konzentration der Verbindung (I) in der Reaktionszone derartig gesunken ist, dass eine effektive destillative Abtrennung, d. h. in ausreichender Menge und Reinheit, in technisch sinnvoller Weise nicht mehr möglich ist.

Gegebenenfalls umfasst die Umsetzung in Schritt b) eine Phase (optionale Phase 4), in der die destillative Abtrennung der Verbindung (I) aus dem Reaktionsgemisch unterbrochen wird. Eine solche Phase kann dazu dienen, der Reaktionszone Komponenten, wie frisches Cyclopentadecan-1,5-diol (II), frisches Lösungsmittel oder frischen Katalysator, zuzuführen und/oder um den Gehalt der Verbindung (I) in der Reaktionszone nach einer Phase der Abtrennung wieder zu erhöhen.

An die Phase 4 kann sich erneut eine Phase 3 anschließen, wobei die Phasen 3 und 4 prinzipiell beliebig oft nacheinander durchlaufen werden können.

Im einfachsten Fall besteht die Destillationszone (d. h. die erfindungsgemäß eingesetzten Destillationsvorrichtung) aus einer Vorrichtung zur einstufigen (einfachen) Destillation. Geeignete Vorrichtungen zur einstufigen Destillation sind dem Fachmann bekannt. In einer solchen Vorrichtung findet im Wesentlichen kein Stoffaustausch zwischen Brüden und Kondensat statt. Mit anderen Worten erfolgt die einfache Destillation ohne eine Rektifikation. In dieser Ausführungsform wird in Schritt b) vorzugsweise ein Lösungsmittel eingesetzt, das einen Dampfdruck aufweist, der niedriger ist als der Dampfdruck des Cyclopentadecan-1,5-diols (II). Der Dampfdruck bezieht sich dabei auf die Temperatur, die unter den Reaktionsbedingungen in Schritt b) herrscht. Bevorzugt ist das Lösungsmittel dann ausgewählt unter Polyalkylenglycolen und deren Mono- und Dialkylethern. Durch den Zusatz des Lösungsmittels wird insbesondere eine bessere Durchmischung der Reaktionszone gewährleistet. Alternativ wird ein Lösungsmittel eingesetzt, das einen Dampfdruck aufweist, der zwischen dem Dampfdruck der Verbindung (I) und dem Dampfdruck der Verbindung (II) liegt. Speziell dient das erfindungsgemäße Verfahren zur Herstellung von 14-Methyl-16-oxabicyclo[10.3.1]-pentadecen (I.1) aus 3-Methyl-cyclopentadecan-1,5-diol (II.1). Dann wird bevorzugt ein Lösungsmittel eingesetzt, das einen Dampfdruck aufweist, der zwischen dem Dampfdruck des 14-Methyl-16-oxabicyclo[10.3.1]pentadecens (I.1) und dem Dampfdruck des 3-Methyl-cyclopentadecan-1,5-diols liegt. Dadurch kann die Temperatur niedrig gehalten werden. Des Weiteren kann so der Gehalt der Verbindung (I) in der Reaktionszone einfacher auf niedrigem Niveau gehalten werden. Das Lösungsmittel kann dann anschließend von der Verbindung (I) getrennt werden, z. B. destillativ.

Das durch einstufige Destillation in Schritt c) abgetrennte Produkt weist vorzugsweise einen Gehalt an der Verbindung der allgemeinen Formel (I) von 75 bis 95 Gew.-%, bezogen auf das Gesamtgewicht des abgetrennten Produkts auf.

Speziell dient das erfindungsgemäße Verfahren zur Herstellung von 14-Methyl-16-oxa-bicyclo[10.3.1]pentadecen (I.1) aus 3-Methyl-cyclopentadecan-1,5-diol (II.1). Dann weist das durch einstufige Destillation in Schritt c) abgetrennte Produkt vorzugsweise einen Gehalt an 14-Methyl-16-oxabicyclo[10.3.1]pentadecen (I.1) von 75 bis 95 Gew.-%, bezogen auf das Gesamtgewicht des abgetrennten Produkts auf.

In einer typischen Zusammensetzung zur Herstellung von 14-Methyl-16-oxa-bicyclo[10.3.1]pentadecen (I.1) aus 3-Methyl-cyclopentadecan-1,5-diol (II.1) enthält das durch einstufige Destillation in Schritt c) abgetrennte Produkt die folgenden Verbindungen, jeweils bezogen auf das Gesamtgewicht des abgetrennten Produkts:
14-Methyl-16-oxabicyclo[10.3.1]pentadecen (1.1): 75 - 95 Gew.-%,
3-Methyl-cyclopentadecan-1,5-diol (II.1): 0 - 5 Gew.-%,
3-Methylcyclopentadecan-1,5-dion (III.1): 1 - 10 Gew.-%,
14-Methyl-16-oxabicyclo[10.3.1]hexadecan (VI.1): 0 - 15 Gew.-%.

Das durch einstufige Destillation in Schritt c) abgetrennte Produkt kann gewünschtenfalls einer weiteren Aufarbeitung unterzogen werden. Bevorzugt wird das durch einstufige Destillation in Schritt c) abgetrennte Produkt einer weiteren Destillation unter Erhalt wenigstens einer an der Verbindung (I) (speziell dem 14-Methyl-16-oxabicyclo[10.3.1]-pentadecen (I.1)) angereicherten Fraktion und wenigstens einer an der Verbindung (I) (speziell dem 14-Methyl-16-oxabicyclo[10.3.1]pentadecen (1.1)) abgereicherten Fraktion unterzogen. Geeignete Destillationsvorrichtungen sind die in Schritt a) zur destillativen Auftrennung des Reaktionsgemischs aus der Hydrierung der Verbindung (III) genannten.

Zur destillativen Abtrennung der bei der Umsetzung in Schritt b) erhaltenen Verbindung (I) (speziell des 14-Methyl-16-oxabicyclo[10.3.1]pentadecens (I.1)) eignen sich in der Regel alle Vorrichtungen zur destillativen Auftrennung von Reaktionsgemischen, die flüssige Komponenten enthalten. Geeignete Vorrichtungen umfassen Destillationskolonnen, wie Bodenkolonnen, die mit Glockenböden, Siebplatten, Siebböden, Packungen oder Füllkörpern ausgerüstet sein können, Drehbandkolonnen, Verdampfer, wie Dünnschichtverdampfer, Fallfilmverdampfer, Zwangsumlaufverdampfer, Sambay-Verdampfer. und Kombinationen davon. Geeignete Packungen und Füllkörper sind z. B. Blech- oder Gewebepackungen, wie Sulzer Mellapak®, Sulzer BX, Montz B1 oder Montz A3 oder Kühni Rombopak, oder regellose Schüttungen von Füllkörpern, wie z. B. Dixon-Ringen, Raschig-Ringen, High-Flow-Ringen oder Raschig-Super-Ringen.

Besonders bevorzugt wird zur destillativen Abtrennung der bei der Umsetzung in Schritt b) erhaltenen Verbindung (I) (speziell des 14-Methyl-16-oxabicyclo[10.3.1]-pentadecens (1.1)) wenigstens eine Destillationskolonne verwendet. Insbesondere wird zur destillativen Abtrennung der bei der Umsetzung in Schritt b) erhaltenen Verbindung (I) (speziell des 14-Methyl-16-oxabicyclo[10.3.1]pentadecens (1.1)) wenigstens eine Destillationskolonne verwendet, die wenigstens 10 theoretische Böden aufweist. Die zur destillativen Abtrennung eingesetzte Destillationskolonne steht in der Regel in direkter Verbindung mit der Reaktionszone, z. B. einem Rührreaktor. Vorzugsweise wird zur Umsetzung in Schritt b) und zur destillativen Abtrennung der Verbindung (I) (speziell des 14-Methyl-16-oxabicyclo[10.3.1]pentadecens (1.1)) in Schritt c) ein Rührreaktor eingesetzt, an dem eine Destillationskolonne installiert ist. Der Rührreaktor fungiert somit prinzipiell als beheizter Sumpf für die Destillationskolonne. Im Falle der Verwendung mehrerer in Serie geschalteter Reaktoren, kann jeder dieser Reaktoren mit einer Destillationskolonne ausgerüstet sein oder es kann, vorzugsweise aus dem in Stromrichtung letzten Kesseln der Reaktorkaskade, der die Verbindung (I) enthaltende Dampf über eine oder mehrere Leitungen einer Destillationskolonne zugeführt werden.

Zur destillativen Abtrennung der bei der Umsetzung in Schritt b) erhaltenen Verbindung (I) (speziell des 14-Methyl-16-oxabicyclo[10.3.1]pentadecens (1.1)) unter Verwendung wenigstens einer Destillationskolonne weist das erfindungsgemäß verwendete Lösungsmittel vorzugsweise einen Dampfdruck auf, der zwischen dem Dampfdruck der Verbindung (I) und dem Dampfdruck der Verbindung (II) liegt. Besonders bevorzugt weist das erfindungsgemäß verwendete Lösungsmittel einen Dampfdruck auf, der zwischen dem Dampfdruck der Verbindung (I) und dem Dampfdruck der Verbindung (III) liegt. Auf die zuvor für den Einsatz in Schritt b) genannten geeigneten und bevorzugten Lösungsmittel wird in vollem Umfang Bezug genommen.

Bevorzugt wird die bei der Umsetzung in Schritt b) freigesetzte Verbindung (I) (speziell 14-Methyl-16-oxabicyclo[10.3.1]pentadecen (I.1)) absatzweise oder kontinuierlich aus dem Reaktionsgemisch abgetrennt.

In einer speziellen Ausführungsform wird der Reaktionszone kontinuierlich eine Verbindung (II) (speziell 3-Methyl-cyclopentadecan-1,5-diol (11.1)) zugeführt und wird die freigesetzte Verbindung (I) (speziell 14-Methyl-16-oxabicyclo[10.3.1]pentadecen (I.1)) kontinuierlich aus dem Reaktionsgemisch abgetrennt.

Das durch fraktionierte Destillation in Schritt c) abgetrennte Produkt weist vorzugsweise einen Gehalt an Verbindung (I) (speziell an 14-Methyl-16-oxabicyclo[10.3.1]pentadecen (1.1)) von 80 bis 99 Gew.-%, besonders bevorzugt von 85 bis 99 Gew.-%, bezogen auf das Gesamtgewicht des abgetrennten Produkts auf.

In einer typischen Zusammensetzung zur Herstellung von 14-Methyl-16-oxa-bicyclo[10.3.1]pentadecen (I.1) aus 3-Methyl-cyclopentadecan-1,5-diol (II.1) enthält das durch fraktionierte Destillation in Schritt c) abgetrennte Produkt die folgenden Verbindungen, jeweils bezogen auf das Gesamtgewicht des abgetrennten Produkts:
- 14-Methyl-16-oxabicyclo[10.3.1]pentadecen (1.1): 80 - 99 Gew.-%,
- 3-Methyl-cyclopentadecan-1,5-diol (II.1): 0 - 5 Gew.-%,
- 3-Methylcyclopentadecan-1,5-dion (III.1): 0 - 5 Gew.-%, bevorzugt 0 bis 1 Gew.-%,
- 14-Methyl-16-Oxabicyclo[10.3.1]hexadecan (VI.1): 0 - 15 Gew.-%, bevorzugt 0 bis 10 Gew.-%,
- Lösungsmittel: 0 - 5 Gew.-%, bevorzugt 0 bis 1 Gew.-%,
- 3-Methyl-Cyclopentadecan-5-ol-1-on (VII.1): 0 - 5 Gew.-%.

Andere Zusammensetzungen des Reaktionsgemischs lassen sich in Abhängigkeit von den gewählten Reaktionsbedingungen ebenfalls erzielen.

Bei der destillativen Abtrennung in Schritt c) der bei der Umsetzung in Schritt b) erhaltenen Verbindung (I) (speziell 14-Methyl-16-oxabicyclo[10.3.1]pentadecen (I.1)) wird zunächst ein Dampf abgezogen, der anschließend zumindest teilweise kondensiert wird. Zur Kondensation bzw. partiellen Kondensation des Dampfs können alle geeigneten Kondensatoren verwendet werden. Diese können mit beliebigen Kühlmedien gekühlt werden. Kondensatoren mit Luftkühlung und/oder Wasserkühlung sind bevorzugt.

Der Kondensator befindet sich in der Regel am Kopf, d. h. am oberen Ende der Destillationskolonne, oder ist im Kopf der Kolonne integriert.

Unter dem Begriffen "Kopf der Kolonne" oder "Kollonnenkopf" wird im Rahmen der vorliegenden Erfindung der Bereich einer Destillationskolonne verstanden, der sich am oberen Ende, d. h. in der Regel im oberen Fünftel, bevorzugt im oberen Zehntel, der Destillationskolonne befindet.

In der Regel liegt das Verhältnis von abgetrenntem Strom zu in die Kolonne zurückgeführtem Strom im Bereich von 1 : 1 bis 1 : 30, ganz speziell im Bereich von 1 : 1 bis 1 : 20.

Üblicherweise wird mit der Abtrennung der bei der Umsetzung in Schritt b) erhaltenen Verbindung (I) (speziell des 14-Methyl-16-oxabicyclo[10.3.1]pentadecens (1.1)) begonnen, sobald sich die Temperatur am Kolonnenkopf nach Beginn der Umsetzung in Schritt b) nicht mehr wesentlich ändert. Dies ist nach wenigen Minuten bis wenigen Stunden der Fall.

Während der destillativen Abtrennung der bei der Umsetzung in Schritt b) erhaltenen Verbindung (I) (speziell des 14-Methyl-16-oxabicyclo[10.3.1]pentadecens (I.1)) wird das Rücklaufverhältnis, wie oben definiert, bevorzugt so eingestellt, dass die Temperatur am Kolonnenkopf möglichst konstant bleibt. Der Ausdruck "möglichst konstant" bedeutet in diesem Zusammenhang, dass die Temperatur am Kolonnenkopf um weniger als 10 °C, insbesondere um weniger als 5 °C oder 3 °C schwankt. Mit anderen Worten, wird das Rücklaufverhältnis am Kolonnenkopf so eingestellt, dass die Zusammensetzung (Reinheit) des Kopfstroms bezüglich der Verbindung (I) (speziell des 14-Methyl-16-oxabicyclo[10.3.1]pentadecens (1.1)) im Wesentlichen konstant bleibt.

In einer bevorzugten Ausführungsform des erfindungsgemäßen Verfahrens wird zur Umsetzung in Schritt b) zusätzlich wenigstens eine Verbindung (II) (speziell 3-Methyl-cyclopentadecan-1,5-diol (II.1)) zugeführt. Die Zuführung der wenigstens einen Verbindung (II) zur Umsetzung in Schritt b) kann stufenweise oder kontinuierlich, bevorzugt kontinuierlich über den gesamten Verlauf der Umsetzung erfolgen. Durch die Zuführung soll der durch die destillative Ausschleusung der Verbindung (I) (speziell des 14-Methyl-16-oxabicyclo[10.3.1]pentadecens (1.1)) bedingte Verlust an Verbindung (II) im Reaktionsgemisch, ausgeglichen werden. Bevorzugt erfolgt die Zuführung der wenigstens einen Verbindung (II) in der Weise, dass die Menge an Verbindung (II) im Reaktionsgemisch während der destillativen Ausschleusung der Verbindung (I) (speziell des 14-Methyl-16-oxabicyclo[10.3.1]pentadecens (I.1)) möglichst konstant bleibt.

### Schritt d):

In einer speziellen Ausführungsform des erfindungsgemäßen Verfahrens werden die Verbindungen der allgemeinen Formel (I) einer weiteren Umsetzung unter Erhalt wenigstens einer Verbindung der allgemeinen Formel (IV) unterzogen 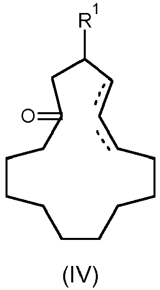 worin das Symbol ------ einmal für eine Einfachbindung und einmal für eine Doppelbindung steht und R¹ für Wasserstoff oder C₁-C₄-Alkyl steht.

In einer speziellen Ausführung steht R¹ für Methyl.

### Schritt d):

In einer weiteren speziellen Ausführungsform des erfindungsgemäßen Verfahrens werden die Verbindungen der allgemeinen Formel (IV) einer weiteren Umsetzung unter Erhalt wenigstens einer Verbindung der allgemeinen Formel (V) unterzogen 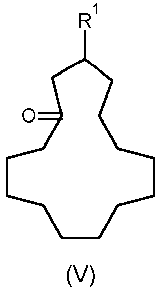 worin R¹ für Wasserstoff oder C₁-C₄-Alkyl steht.

In einer speziellen Ausführung steht R¹ für Methyl.

### FIGURENBESCHREIBUNG

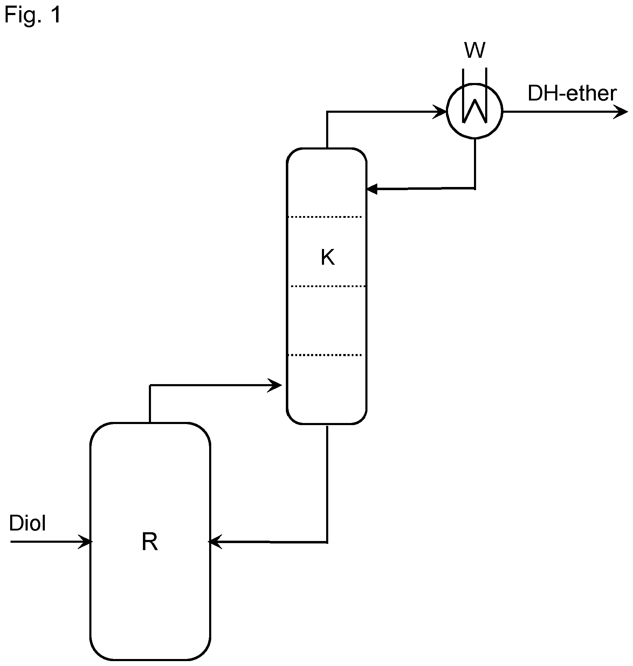

Figur 1 zeigt eine Vorrichtung, die prinzipiell zur kontinuierlichen, semikontinuierlichen (semi-batch) oder diskontinuierlichen (batch) Durchführung des erfindungsgemäßen Verfahrens geeignet ist. In den Reaktor R wird 3-Methyl-cyclopentadecan-1,5-diol eingespeist und in Gegenwart eines heterogenen Katalysators umgesetzt. Bei diskontinuierlicher Fahrweise erfolgt die Zugabe des 3-Methyl-cyclopentadecan-1,5-diols vor Beginn der Umsetzung. Gegebenenfalls kann nach Abnahme des 3-Methyl-cyclopentadecan-1,5-diolgehalts im Reaktor R unter einen bestimmten Grenzwert frisches 3-Methyl-cyclopentadecan-1,5-diol in den Reaktor R eingespeist werden. Dies kann sowohl einmalig als auch mehrmals erfolgen. Bei kontinuierlicher Fahrweise erfolgt die Zugabe des 3-Methyl-cyclopentadecan-1,5-diols nach Maßgabe seines Verbrauchs zur Herstellung von 14-Methyl-16-oxabicyclo[10.3.1]pentadecen. Der im Reaktor R gebildete 14-Methyl-16-oxabicyclo[10.3.1]pentadecen wird über die Kolonne K destillativ abgetrennt und in einem mit dem Wärmetauscher W verbundenen Kondensator kondensiert. Es ist auch eine semikontinuierliche Fahrweise möglich, bei der einer der Schritte, Zugabe des 3-Methyl-cyclopentadecan-1,5-diols oder die Abtrennung des 14-Methyl-16-oxabicyclo[10.3.1]pentadecens, kontinuierlich erfolgt und der andere diskontinuierlich.

Die folgenden Beispiele dienen der Verdeutlichung der Erfindung, ohne sie in irgendeiner Weise zu beschränken.

### BEISPIELE

### Liste der Verbindungen:

- 14-Methyl-16-oxabicyclo[10.3.1]pentadecen (I.1),
- 3-Methyl-cyclopentadecan-1,5-diol (II.1),
- 3-Methylcyclopentadecan-1,5-dion (III.1),
- 14-Methyl-16-Oxabicyclo[10.3.1]hexadecan (VI.1),
- 3-Methyl-Cyclopentadecan-5-ol-1-on (VII.1).

Gaschromatographische Analysen wurden nach der folgenden Methode durchgeführt:
GC-System: Agilent 7890 Series A
Säule: DB WAX 30 m (Länge) x 0,32 mm (Innendurchmesser);
FD 0,25 µm (Film);
Injektortemperatur: 230 °C; Detektortemperatur 280 °C; Flussrate: 1,5 ml
Temperaturprogramm: Anfangstemp.: 80 °C auf 250 °C, mit 3 °C/min,
   250 °C, 15 Minuten isotherm.

Die in den vermessenen Proben enthaltenen Verbindungen können verschiedene Isomere aufweisen, z. B. bezüglich der Stellung der Substituenten am Ringsystem (cis,trans-Isomerie) und der Stellung der Substituenten an den Doppelbindungen. Soweit diese Isomere unterschiedliche Retentionszeiten aufwiesen, wurde zur Bestimmung der Menge der zugehörigen Verbindung die Summe aller bestimmbaren Flächenintegrale gebildet. Die Retentionszeiten sind im Folgenden angegeben.

### Beispiel 1:

### (Vergleich, Reaktion ohne zugesetztes Lösungsmittel)

4,0 g Katalysatorsuspension (Raney-Kupfer, 30 % in Wasser) wurden mit 15,08 g 3-Methyl-cyclopentadecan-1,5-diol (II.1) (84,7 GC-FI.%) in einem 100 ml Dreihalskolben vorgelegt. Der Druck wurde zunächst bei Raumtemperatur auf 220 mbar abgesenkt. Dann wurde das Reaktionsgemisch von Raumtemperatur auf 166 °C aufgeheizt und gleichzeitig der Druck von 220 mbar auf 40 mbar abgesenkt, wobei der größte Teil des Wassers der Katalysatorsuspension sowie des Methanols destillierte. Nach Temperaturerhöhung auf 172 bis 176 °C und Druckverringerung auf 1 bis 2 mbar wurde der Ansatz weitere 5 h gerührt. Im Anschluss wurde die Temperatur auf 180 °C erhöht, und es wurde über 12 h einstufig (ohne Rektifikation) Destillat abgenommen. Die Kopftemperatur betrug dabei am Anfang der Destillatabnahme 165 °C und erhöhte sich im Verlauf der Destillation auf 175 °C. Insgesamt wurden 10 g Destillat erhalten. Der Gehalt an 14-Methyl-16-oxabicyclo[10.3.1]pentadecen (I.1) im Destillat beträgt 10,6 %, der des 3-Methylcyclopentadecan-1,5-dions (III.1) 27,7 % und der des 3-Methyl-cyclopentadecan-1,5-diols (II.1) 8,3 %. Das entspricht einer Ausbeute von 9 %.

### Beispiel 2:

### (erfindungsgemäß)

3 g Katalysatorsuspension (Raney-Kupfer, 50 % in Wasser) wurden dreimal mit Methanol gewaschen. Anschließend wurden 10 g 3-Methyl-cyclopentadecan-1,5-diol (II.1) (82,5 GC Gew.-%) in 20 g Polyethylenglycol (ca. 8 PEG-Einheiten, Lutrol ® E400 der BASF SE, Dampfdruck bei 180 °C: 0,02 mbar) zusammen mit dem gewaschenen Katalysator bei Raumtemperatur in einem 100 ml Dreihalskolben vorgelegt. Das Methanol und das restliche Wasser wurden bei 50 °C bei einem Druck von 250 bis 3 mbar langsam abdestilliert. Die Reaktionsmischung wurde danach bei einem Druck von 20 mbar auf 200 °C aufgeheizt. Die Temperatur wurde für 16 Stunden gehalten. Anschließend wurde der Druck auf 1 mbar abgesenkt und die leichtsiedenden Komponenten einstufig abdestilliert. Während des gesamten Versuchs wurde mit einem Magnetrührer für eine gute Durchmischung gesorgt. Es konnten 4,5 g Destillat mit einem 14-Methyl-16-oxabicyclo[10.3.1]pentadecen-Gehalt von 87,2 GC-Flächen-% gewonnen werden, was einer Ausbeute von 52 % entspricht. Der 3-Methyl-cyclopentadecan-1,5-diolgehalt betrug 0,8 GC-Flächen-% und der 3-Methylcyclopentadecan-1,5-diongehalt 3,9 GC-Flächen-%.

### Beispiel 3:

### (erfindungsgemäß, Reaktionsgemisch verdünnt mit schwersiedendem Lösungsmittel, das zwischen 14-Methyl-16-oxabicyclo[10.3.1]pentadecen und 3-Methylcyclopentadecan-1,5-dion siedet)

0,25 g Katalysatorsuspension (Raney-Kupfer aktiv, 50 % in Wasser) wurden dreimal mit Methanol gewaschen. Anschließend wurden 5 g 3-Methyl-cyclopentadecan-1,5-diol (II.1) (82,7 GC-FI.%) in 10 g 1-Hexadecanol (Dampfdruck bei 180 °C: 11 mbar) zusammen mit dem gewaschenen Katalysator bei Raumtemperatur in einem 100 ml Dreihalskolben vorgelegt. Während des gesamten Versuchs wurde mit einem Magnetrührer für eine gute Durchmischung gesorgt. Die Temperatur wurde auf zunächst auf 70 °C erhöht, um das 1-Hexadecanol zu schmelzen. Das Methanol und das restliche Wasser wurden bei 70 °C langsam abdestilliert. Die Reaktionsmischung wurde danach bei einem Druck von 40 mbar auf 180 °C unter ständigem Rühren aufgeheizt. Die Temperatur wurde für 20 Stunden gehalten. Es wurde jeweils bei 1, 3, 5 und 20 h eine Probe gezogen und per GC analysiert. In der folgenden Tabelle 1 sind die Gehalte an dem Produkt 14-Methyl-16-oxabicyclo[10.3.1]pentadecen (I.1) an dem eingesetzten 3-Methyl-cyclopentadecan-1,5-diol (II.1) sowie der Verbindungen (III.1), (VI.1) und (VII.1) in Abhängigkeit von der Reaktionsdauer als GC-Flächen-% (GC-FI.%) (ohne Berücksichtigung des 1-Hexadecanols) angegeben.

**Tabelle 1**

| Retentionszeit [t/min] | 22,75/22,95 | 20,7/27 | 40,5 | 43,5/44,8 | 50,8-52 |
|---|---|---|---|---|---|
| | (I.1) [GC-FI.%] | (VI.1) [GC-FI.%] | (III.1) [GC-FI.%] | (VII.1) [GC-FI.%] | (II.1) [GC-FI.%] |
| Edukt (0h) | 0,0 | 0,0 | 0,0 | 0 | 98,6 |
| 1h | 10,2 | 1,3 | 1,3 | 7,2 | 73,5 |
| 3h | 52,2 | 5,6 | 7,3 | 13,18 | 17,3 |
| 5h | 63,2 | 8,5 | 8,0 | 8,28 | 6,5 |
| 20h | 66,2 | 19,6 | 7,1 | 0 | 1,1 |

Anschließend wurde der Druck auf 1 mbar und die Sumpftemperatur auf 124 bis 140 °C (siehe Tabelle) abgesenkt und die leichtsiedenden Komponenten einstufig abdestilliert. Es konnten drei Fraktionen Destillat (Fr1: 0,6 g, Fr2: 1,3 g, Fr3: 7,3 g) abgezogen werden. Alle drei Fraktionen waren fest und weiß.

**Tabelle 2**

| Destillat | Sumpf -temp. °C | Kopftemp. °C | Druck mbar | (I.1) [GC-FI.%] | (VI.1) [GC-FI.%] | 1-Hexadecanol [GC-FI.%] | (III.1) [GC-FI.%] | (II.1) [GC-FI.%] |
|---|---|---|---|---|---|---|---|---|
| Fr1 | 124-127 | 111-114 | 1 | 62,25 | 4,7 | 22,88 | 0,87 | 1,03 |
| Fr2 | 127-129 | 112-116 | 1 | 59,4 | 5,33 | 26,22 | 1,03 | 0 |
| Fr3 | 128-140 | 116-128 | 1 | 15,79 | 4,16 | 75,76 | 2,73 | 0,34 |

Beispiel 3 wurde wiederholt unter Einsatz von Nonadecan (Dampfdruck bei 180 °C: 11 mbar), Tetradecanol (Dampfdruck bei 180 °C: 34 mbar), Heptadecanol (Dampfdruck bei 180 °C: 7 mbar) und Octadecanol (Dampfdruck bei 180 °C: 4 mbar) als Lösungsmittel. Es konnten in jedem Falle Produktfraktionen mit hohem 14-Methyl-16-oxabicyclo[10.3.1]pentadecengehalt isoliert werden.

### Beispiel 4:

### (erfindungsgemäß, Reaktionsgemisch verdünnt mit Hexadecanol, das zwischen 14-Methyl-16-oxabicyclo[10.3.1]pentadecen (I.1) und 3-Methylcyclopentadecan-1,5-dion (III.1) siedet, 14-Methyl-16-oxabicyclo[10.3.1]pentadecen wurde stufenweise zusammen mit Hexadecanol abdestilliert, 3-Methyl-cyclopentadecan-1,5-diol (II.1) wurde mit Hexadecanol nachgeführt)

0,5 g Katalysatorsuspension (Raney-Kupfer aktiv, 50 % in Wasser) wurden dreimal mit Methanol gewaschen. Anschließend wurden 10 g 3-Methyl-cyclopentadecan-1,5-diol (II.1) (85,1 GC-FI.%) in 20 g 1-Hexadecanol (Dampfdruck bei 180 °C: 11 mbar) zusammen mit dem gewaschenen Katalysator bei Raumtemperatur in einem 100 ml Dreihalskolben vorgelegt. Die Temperatur wurde auf zunächst auf 70 °C erhöht, um das 1-Hexadecanol zu schmelzen. Das Methanol und das restliche Wasser wurden bei 70 °C langsam abdestilliert. Die Reaktionsmischung wurde danach bei einem Druck von 40 mbar auf 180 °C aufgeheizt. Die Temperatur wurde für 2 h gehalten. Danach wurde die Temperatur auf 145 °C und der Druck auf 3 mbar abgesenkt und einstufig 7 g (Fraktion 1) abdestilliert. Das Destillat wurde analysiert und auf Basis des abgezogenen 14-Methyl-16-oxabicyclo[10.3.1]pentadecens (I.1) wurde 3-Methyl-cyclopentadecan-1,5-diol (11.1), zusammen mit 1-Hexadecanol, dem Reaktor zugeführt, sodass sich ein 3-Methyl-cyclopentadecan-1,5-diol / 1-Hexadecanol-Verhältnis wie in der Ausgangsreaktionsmischung ergab. Nun wurde die Temperatur wieder auf 180 °C und der Druck auf 40 mbar angehoben und für 2 h gehalten. Im Anschluss wurden der Druck und die Temperatur erneut auf die oben genannten Werte abgesenkt und 4,5 g (Fraktion 2) sowie 4,8 g (Fraktion 3) destilliert. Erneut wurden 3-Methyl-cyclopentadecan-1,5-diol (11.1) und 1-Hexadecanol dem Reaktor zugeführt. Mit dem gleichen Vorgehen wie oben beschrieben wurde noch eine vierte Fraktion (Fraktion 4) mit 10,8 g erzeugt. Nach der Destillation von Fraktion 4 wurde die Reaktionsmischung, ohne erneut 3-Methyl-cyclopentadecan-1,5-diol und 1-Hexadecanol zuzugeben, für weitere 24 h bei 180 °C und 40 mbar gehalten und schließlich 5,1 g Destillat (Fraktion 5) abdestilliert.

**Tabelle 3**

| | (I.1) [GC-FI.%] | (VI.1) [GC-FI.%] | 1-Hexadecanol [GC-FI.%] | (III.1) [GC-FI.%] | (VII.1) [GC-FI.%] | (11.1) [GC-FI.%] |
|---|---|---|---|---|---|---|
| Retentionszeit | 22,5/22,6 | 26,7 | 34,1 | 40,1 | 43,2/44,5 | 50,8-52 |
| a) | 0,0 | 0,0 | 0,0 | 0,0 | 0,0 | 95,3 |
| b) | 22,46 | 2,89 | nicht integriert | 9,68 | 25,59 | 28,12 |
| c) | 20,1 | 1,6 | 70,2 | 1,9 | 2,92 | 3,1 |
| d) | 15,1 | 4,7 | nicht integriert | 12,2 | 28,03 | 27,5 |
| e) | 12,3 | 3,0 | nicht integriert | 8,2 | 15,74 | 43,9 |
| f) | 39,4 | 4,3 | 42,5 | 1,5 | 0,8 | 1,4 |
| g) | 10,8 | 3,7 | 79,8 | 2,3 | 1,22 | 0,3 |
| h) | 11,2 | 4,4 | nicht integriert | 10,9 | 14,37 | 51,3 |
| i) | 5,9 | 2,5 | nicht integriert | 6,4 | 8,07 | 68,8 |
| k) | 10,6 | 3,0 | 81,9 | 1,2 | 0,36 | 2,7 |
| l) | 5,4 | 2,4 | nicht integriert | 5,3 | 3 | 79,2 |
| m) | 47,1 | 3,1 | 48,4 | 0,0 | 0 | 0,5 |
| n) | 40,7 | 14,1 | nicht Integriert | 13,6 | 0 | 18,2 |

| | | | | | | |
|---|---|---|---|---|---|---|
| a) Edukt (t = 0) b) Sumpf vor Abzug Fraktion 1 c) Fraktion 1 d) Sumpf nach Abzug Fraktion 1 e) Sumpf nach Zugabe 3-Methylcyclopentadecan-1,5-dion (II.1)/1-Hexadecanol f) Fraktion 2 g) Fraktion 3 h) Sumpf nach Abzug Fraktion 3 i) Sumpf nach Zugabe 3-Methylcyclopentadecan-1,5-dion (II.1)/1-Hexadecanol k) Fraktion 4 l) Sumpf nach Abzug Fraktion 4 m) Fraktion 5 n) Sumpf nach Abzug Fraktion 5 | | | | | | |

### Beispiel 5:

### (erfindungsgemäß, Reaktionsgemisch verdünnt mit Lösungsmittel, das zwischen 14-Methyl-16-oxabicyclo[10.3.1]pentadecen (II.1) und 3-Methylcyclopentadecan-1,5-dion (III.1) siedet sowie Abzug des 14-Methyl-16-oxabicyclo[10.3.1]pentadecens (I.1) über eine Kolonne)

1,5 g Katalysatorsuspension (Raney-Kupfer aktiv, 50 % in Wasser) wurden dreimal mit Methanol gewaschen. Anschließend wurden 30 g 3-Methyl-cyclopentadecan-1,5-diol (II.1) (95,2 GC-FI.%) in 60 g 1-Hexadecanol (Dampfdruck bei 180 °C: 11 mbar) zusammen mit dem gewaschenen Katalysator bei Raumtemperatur in einem 100 ml Dreihalskolben vorgelegt. Dem Kolben war eine Füllkörperkolonne aufgesetzt (Betthöhe Füllkörper 53 cm, Füllkörper 3 mm Raschigringe, Innendurchmesser Kolonne: 1,5 cm). Die Temperatur wurde auf zunächst auf 70 °C erhöht, um das 1-Hexadecanol zu schmelzen. Das Methanol und das restliche Wasser wurden bei 70 °C langsam abdestilliert. Anschließend wurde das Reaktionsgemisch bei 3 mbar Kopfdruck auf 181 °C unter Durchmischung durch einen Magnetrührer erwärmt. Nach 1 h stellte sich ein Destillatfluss ein, wobei das Destillat zunächst unter totalem Rücklauf bei 2 bis 3 mbar Kopfdruck, im Kopf der Kolonne für 1 h angesammelt wurde. Über die nächsten 2,5 h wurden insgesamt 10,6 g Destillat (Fraktion 1, Fr1) abgenommen. Unter konstanten Bedingungen wurden jeweils nach weiteren 3 h und 6 h weitere Fraktionen gezogen (Fraktion 2, Fr2: 10,0 g, Fraktion 3, Fr3: 3,9 g). Der Hexadecanol-Gehalt aller Fraktionen lag unter 0,5 %. Der Gehalt an 14-Methyl-16-oxabicyclo[10.3.1]pentadecen (I.1) war 92 % in Fraktion 1,87 % in Fraktion 2 und 57,8 % in Fraktion 3. In der Summe entspricht das einer Ausbeute von 80 %. In keiner der Fraktionen konnte 3-Methylcyclopentadecan-1,5-dion nachgewiesen werden. Die Hauptnebenkomponente im Destillat war Ether.

**Tabelle 4**

| | (I.1) [GC-FI.%] | (VI.1) [GC-FI.%] | 1-Hexadecanol [GC-FI.%] |
|---|---|---|---|
| Retentionszeit | 22,7/22,9 | 20,6 | 33,8 |
| Fraktion 1 | 92 | 2,6 | 0,4 |
| Fraktion 2 | 87 | 9,8 | 0,3 |
| Fraktion 3 | 57,8 | 42,3 | 0,4 |

### Beispiel 6:

### (erfindungsgemäß, Reaktionsgemisch verdünnt mit Lösungsmittel, das zwischen 14-Methyl-16-oxabicyclo[10.3.1]pentadecen (I.1) und 3-Methylcyclopentadecan-1,5-dion (III.1) siedet, kontinuierliche Reaktionsführung unter Zuführung von sowie kontinuierlicher Abzug des 14-Methyl-16-oxabicyclo[10.3.1]pentadecens (I.1) über eine Kolonne)

2,25 g Katalysator (Raney-Kupfer aktiv, abgesetzt in Wasser, Entnahme aus Festbett) wurden dreimal mit Methanol gewaschen. Anschließend wurden 90 g 3-Methyl-cyclo-pentadecan-1,5-diol (11.1) (97,1 GC-Gew.-%) in 180 g 1-Hexadecanol (Dampfdruck bei 180 °C: 11 mbar) zusammen mit dem gewaschenen Katalysator bei Raumtemperatur in einem 500 ml Dreihalskolben vorgelegt. Dem Kolben war eine Packungskolonne aufgesetzt (Höhe Packung 60 cm, Packung Montz DN30 A3-1000). Die Temperatur wurde auf zunächst auf 70 °C erhöht, um das 1-Hexadecanol zu schmelzen. Das Methanol und das restliche Wasser wurden bei 70 °C langsam abdestilliert. Anschließend wurde das Reaktionsgemisch bei 3 mbar Kopfdruck auf 180 °C unter Durchmischung durch einen Magnetrührer erwärmt. Nach 1,5 h stellte sich ein Destillatfluss ein, wobei das Destillat zunächst, unter totalem Rücklauf bei 3 mbar Kopfdruck, im Kopf der Kolonne für 1 h angesammelt wurde.

Bei einem mittleren Rücklaufverhältnis von 30 wurden in den folgenden Tagen bei einer Gesamtversuchszeit von 156 h insgesamt 17 Destillatfraktionen abgenommen. Das Rücklaufverhältnis wurde dabei zwischen 15 und 40 variiert, so dass die Kopftemperatur bei 134 °C konstant blieb. Über die Variation des Rücklaufverhältnisses wurde gewährleistet, dass dem Reaktionsgemisch nicht mehr Produkt entzogen wurde als durch die Reaktion gebildet wurde. Die Fraktionen sowie ihre Zusammensetzung kann Tabelle 1 entnommen werden. Durch die ständige Abnahme des 14-Methyl-16-oxa-bicyclo[10.3.1]pentadecens (I.1) wurde auch die Sumpftemperatur bei 180 °C konstant gehalten. Damit das System nicht an 3-Methyl-cyclopentadecan-1,5-diol (II.1) verarmt, wurden so viele Äquivalente 3-Methyl-cyclopentadecan-1,5-diol (II.1) nachgeführt wie an 14-Methyl-16-oxabicyclo[10.3.1]pentadecen (I.1) und Nebenkomponenten abgezogen wurden. Die Zugabe des 3-Methyl-cyclopentadecan-1,5-diols (II.1) erfolge gepulst (portionsweise). Als die Menge an abgenommenem Destillat langsam nachließ, wurden nach 24 und 89 Stunden jeweils weitere 2,25 g Katalysator in den Reaktor gegeben. Die sich im Reaktor einstellenden Konzentrationen können Tabelle 2 entnommen werden. Nach 104 h wurde kein 3-Methyl-cyclopentadecan-1,5-diol (II.1) mehr nachgeführt und das restliche 3-Methyl-cyclopentadecan-1,5-diol (II.1) zu 14-Methyl-16-oxa-bicyclo[10.3.1]pentadecen (I.1) umgesetzt. Über das gesamte Experiment ergibt sich eine Ausbeute von 14-Methyl-16-oxabicyclo[10.3.1]pentadecen (1.1), bezogen auf 3-Methyl-cyclopentadecan-1,5-diol (II.1), von 80 %. Insgesamt wurden 383 g Einsatzmaterial mit einem 3-Methyl-cyclopentadecan-1,5-diolgehalt von 93,7 % zugeführt.

**Tabelle 5: Übersicht über die Fraktionen. Alle Konzentrationsangaben sind GC-FI.%.**

| Fraktion | Zeit [h] | Menge [g] | (I.1) [GC-FI.%] Retentionszeit 25,28 | (VI.1) [GC-FI.%] Retentionszeit 29,18 |
|---|---|---|---|---|
| 1 | 10,8 | 35,9 | 90,7 | 4,3 |
| 2 | 24,5 | 13,3 | 83,2 | 8,1 |
| 3 | 32,0 | 24,2 | 58,9 | 30,9 |
| 4 | 38,0 | 20,9 | 66,4 | 24,1 |
| 5 | 45,5 | 25,4 | 54,0 | 35,8 |
| 6 | 53,5 | 7,8 | 88,3 | 2,7 |
| 7 | 60,5 | 15,5 | 93,1 | 1,3 |
| 8 | 68,5 | 22,2 | 94,4 | 1,2 |
| 9 | 75,2 | 17,9 | 92,0 | 2,0 |
| 10 | 81,8 | 12,9 | 93,3 | 1,6 |
| 11 | 97,0 | 35,4 | 96,0 | 0,5 |
| 12 | 104,3 | 22,1 | 95,4 | 0,6 |
| 13^{a)} | 143,2 | 62,4 | 86,0 | 5,1 |

| | | | | |
|---|---|---|---|---|
| a) = kein 3-Methyl-cyclopentadecan-1,5-diol (II.1) mehr nachgeführt | | | | |

**Tabelle 6: Übersicht Konzentrationen im Reaktor. Alle Angaben sind GC-FI.%.**

| Probe | Zeit [h] | (I.1) [GC-FI.%] Retentionszeit 25,28 | (VI.1) [GC-FI.%] Retentionszeit 29,18 | (III.1) [GC-FI.%] Retentionszeit 42,76 | (II.1) [GC-FI.%] Retentionszeit 53,55 | 1-Hexadecanol [GC-FI.%] Retentionszeit 36,18 |
|---|---|---|---|---|---|---|
| 1 | 10,8 | 0,3 | 1,9 | 1,1 | 28,8 | 66,5 |
| 2 | 24,5 | 0,2 | 3,1 | 1,5 | 13,9 | 79,0 |
| 3 | 32,0 | 0,0 | 2,3 | 1,3 | 30,5 | 63,5 |
| 4 | 38,0 | 0,1 | 1,4 | 1,4 | 26,4 | 66,2 |
| 5 | 53,5 | 1,8 | 3,1 | 1,3 | 35,1 | 52,4 |
| 6 | 75,2 | 1,3 | 3,1 | 1,6 | 30,7 | 54,2 |
| 7 | 89,0 | 7,9 | 1,7 | 1,6 | 29,0 | 48,3 |
| 8 | 130,2 | 3,4 | 4,7 | 2,8 | 17,2 | 53,5 |
| 9 | 155,7 | 0,0 | 5,2 | 5,3 | 1,4 | 50,2 |

## Patentansprüche

1. Verfahren zur Herstellung von Verbindungen der allgemeinen Formel (I) 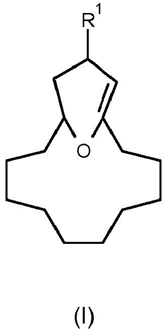 worin R¹ für Wasserstoff oder C₁-C₄-Alkyl steht,
wobei man
a) ein Ausgangsmaterial bereitstellt, das eine Verbindung der allgemeinen Formel II enthält 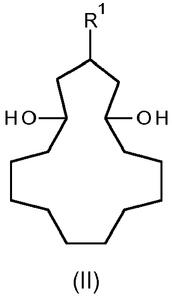
b) das in Schritt a) bereitgestellte Ausgangsmaterial in einer Reaktionszone einer Umsetzung bei einer Temperatur in einem Bereich von 100 bis 240 °C und einem Druck in einem Bereich von 0,1 bis 150 mbar in Gegenwart eines heterogenen Katalysators und eines Lösungsmittels oder eines Lösungsmittelgemisches unterzieht, das bei 180 °C einen Dampfdruck im Bereich von 10⁻⁵ bis 100 mbar aufweist, und
c) aus dem Reaktionsgemisch die Verbindung der Formel (I) destillativ abtrennt.

2. Verfahren nach Anspruch 1, wobei R¹ für Wasserstoff oder Methyl, insbesondere für Methyl, steht.

3. Verfahren nach Anspruch 1 oder 2, wobei die Umsetzung in Schritt b) eine erste Phase umfasst, während der noch keine die Verbindung der Formel (I) enthaltende Fraktion aus dem Reaktionsgemisch destillativ abgetrennt wird.

4. Verfahren nach einem der Ansprüche 1 bis 3, wobei der Dampfdruck des in Schritt b) eingesetzten Lösungsmittels niedriger ist als der Dampfdruck des Diols (II).

5. Verfahren nach einem der Ansprüche 1 bis 3, wobei der Dampfdruck des in Schritt b) eingesetzten Lösungsmittels zwischen dem Dampfdruck der Verbindung (I) und dem Dampfdruck der Verbindung (II) liegt.

6. Verfahren nach einem der Ansprüche 1 bis 3, wobei der Dampfdruck des in Schritt b) eingesetzten Lösungsmittels zwischen dem Dampfdruck der Verbindung (I) und dem Dampfdruck der Verbindung (III) 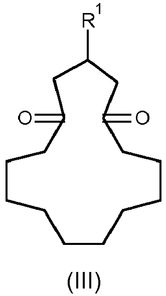 worin R¹ für Wasserstoff oder C₁-C₄-Alkyl steht,
liegt.

7. Verfahren nach einem der vorhergehenden Ansprüche, wobei das in Schritt b) eingesetzte Lösungsmittel ausgewählt ist unter
- aliphatischen, cycloaliphatischen und aromatischen Kohlenwasserstoffen,
- aliphatischen, cycloaliphatischen und aromatischen ein- und mehrwertigen Alkoholen,
- Etheralkoholen, Polyetherpolyolen und deren Mono- und Dialkylethern, aromatischen Ethern und offenkettigen aliphatischen Ethern,
- Ketonen,
- Estern,
- Mischungen davon.

8. Verfahren nach einem der vorhergehenden Ansprüche, wobei das in Schritt b) eingesetzte Lösungsmittel ausgewählt ist unter
- C₁₀-C₃₀-Alkanen,
- C₆-C₃₀-Alkanolen,
- C₂-C₃₀-Alkandiolen,
- Polyalkylenglycolen und deren Mono- und Dialkylethern,
- Mischungen davon.

9. Verfahren nach einem der Ansprüche 1 bis 8, wobei in Schritt c) aus dem Reaktionsgemisch die Verbindung der Formel (I.1) durch einstufige Destillation abgetrennt wird und das abgetrennte Produkt die folgenden Verbindungen, jeweils bezogen auf das Gesamtgewicht des abgetrennten Produkts, enthält:
14-Methyl-16-oxabicyclo[10.3.1]pentadecen (1.1): 75 - 95 Gew.-%,
3-Methyl-cyclopentadecan-1,5-diol (II.1): 0 - 5 Gew.-%,
3-Methylcyclopentadecan-1,5-dion (III.1): 1 - 10 Gew.-%,
14-Methyl-16-Oxabicyclo[10.3.1]hexadecan (VI.1): 0 - 15 Gew.-%.

10. Verfahren nach einem der Ansprüche 1 bis 8, wobei die Abtrennung in Schritt c) eine fraktionierte Destillation umfasst,

11. Verfahren nach Anspruch 10, wobei in Schritt c) zur destillativen Abtrennung einer die Verbindung der Formel (I) enthaltenden Fraktion wenigstens eine Destillationskolonne eingesetzt wird, die wenigstens 10 theoretische Böden aufweist.

12. Verfahren nach Anspruch 10 oder 11 zur Herstellung von 14-Methyl-16-oxa-bicyclo[10.3.1]pentadecen (I.1), wobei in Schritt c) aus dem Reaktionsgemisch die Verbindung der Formel (I) durch fraktionierte Destillation abgetrennt wird und das abgetrennte Produkt die folgenden Verbindungen, jeweils bezogen auf das Gesamtgewicht des abgetrennten Produkts, enthält:
- 14-Methyl-16-oxabicyclo[10.3.1]pentadecen (1.1): 80 - 99 Gew.-%,
- 3-Methyl-cyclopentadecan-1,5-diol (II.1): 0 - 5 Gew.-%,
- 3-Methylcyclopentadecan-1,5-dion (III.1): 0 - 5 Gew.-%, bevorzugt 0 bis 1 Gew.-%,
- 14-Methyl-16-Oxabicyclo[10.3.1]hexadecan (VI.1): 0 - 15 Gew.-%, bevorzugt 0 bis 10 Gew.-%,
- Lösungsmittel: 0 - 5 Gew.-%, bevorzugt 0 bis 1 Gew.-%,
- 3-Methyl-Cyclopentadecan-5-ol-1-on (VII.1): 0 - 5 Gew.-%.

13. Verfahren nach einem der vorhergehenden Ansprüche, wobei in Schritt b) der Gehalt des Reaktionsgemischs an dem Lösungsmittel immer bei mindestens 20 Gew.-%, bevorzugt mindestens 30 Gew.-%, insbesondere mindestens 50 Gew.-%, bezogen auf das Gesamtgewicht des Reaktionsgemischs in der Reaktionszone, gehalten wird.

14. Verfahren zur Herstellung von Folgeprodukten der Verbindung der Formel (I) wie in einem der vorhergehenden Ansprüche definiert, wobei man zusätzlich
d) die Verbindungen der allgemeinen Formel (I) einer Umsetzung unter Erhalt wenigstens einer Verbindung der allgemeinen Formel (IV) unterzieht 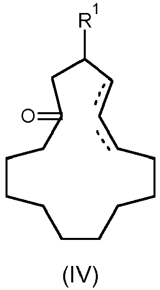 worin das Symbol ------ einmal für eine Einfachbindung und einmal für eine Doppelbindung steht und R¹ für Wasserstoff oder C₁-C₄-Alkyl steht.

15. Verfahren nach Anspruch 14, wobei man zusätzlich
e) die Verbindungen der allgemeinen Formel (IV) einer Hydrierung unter Erhalt der Verbindung der allgemeinen Formel (V) unterzieht 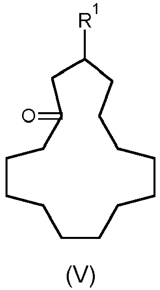 worin R¹ für Wasserstoff oder C₁-C₄-Alkyl steht.

## Claims

1. A method for preparing compounds of the general formula (I) 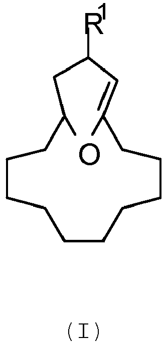 where R¹ is hydrogen or C₁-C₄-alkyl,
wherein
a) a starting material is provided comprising a compound of the general formula II 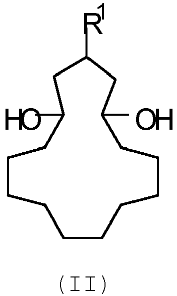
b) the starting material provided in step a) is subjected in a reaction zone to a reaction at a temperature in a range from 100 to 240°C and a pressure in a range from 0.1 to 150 mbar in the presence of a heterogeneous catalyst and a solvent or a solvent mixture having a vapor pressure in the range from 10⁻⁵ to 100 mbar at 180°C, and
c) the compound of formula (I) is separated from the reaction mixture by distillation.

2. The method according to claim 1, wherein R¹ is hydrogen or methyl, particularly methyl.

3. The method according to claim 1 or 2, wherein the reaction in step b) comprises a first phase, during which the fraction not comprising any of the compound of formula (I) is separated from the reaction mixture by distillation.

4. The method according to any of claims 1 to 3, wherein the vapor pressure of the solvent used in step b) is less than the vapor pressure of diol (II) .

5. The method according to any of claims 1 to 3, wherein the vapor pressure of the solvent used in step b) is between the vapor pressure of compound (I) and the vapor pressure of compound (II).

6. The method according to any of claims 1 to 3, wherein the vapor pressure of the solvent used in step b) is between the vapor pressure of compound (I) and the vapor pressure of compound (III) 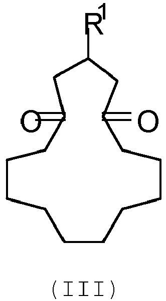 where R¹ is hydrogen or C₁-C₄-alkyl.

7. The method according to any of the preceding claims, wherein the solvent used in step b) is selected from
- aliphatic, cycloaliphatic and aromatic hydrocarbons,
- aliphatic, cycloaliphatic and aromatic monohydric and polyhydric alcohols,
- ether alcohols, polyether polyols and mono- and dialkyl ethers thereof, aromatic ethers and open-chain aliphatic ethers,
- ketones,
- esters,
- mixtures thereof.

8. The method according to any of the preceding claims, wherein the solvent used in step b) is selected from
- C₁₀-C₃₀-alkanes,
- C₆-C₃₀-alkanols,
- C₂-C₃₀-alkanediols,
- polyalkylene glycols and mono- and dialkylethers thereof,
- mixtures thereof.

9. The method according to any of claims 1 to 8, wherein the compound of formula (I.1) is separated from the reaction mixture in step c) by one-stage distillation and the product separated comprises the following compounds, based in each case on the total weight of the separated product:
14-methyl-16-oxabicyclo[10.3.1]pentadecene (I.1): 75 - 95% by weight,
3-methylcyclopentadecane-1,5-diol (II.1): 0 - 5% by weight,
3-methylcyclopentadecane-1,5-dione (III.1): 1 - 10% by weight,
14-methyl-16-oxabicyclo[10.3.1]hexadecane (VI.1): 0 - 15% by weight.

10. The method according to any of claims 1 to 8, wherein the separation in step c) comprises a fractional distillation.

11. The method according to claim 10, wherein at least one distillation column having at least 10 theoretical plates is used for the separation by distillation of a fraction comprising the compound of formula (I) in step c).

12. The method according to claim 10 or 11 for preparing 14-methyl-16-oxabicyclo[10.3.1]pentadecene (I.1), wherein the compound of formula (I) is separated from the reaction mixture in step c) by fractional distillation and the product separated comprises the following compounds, based in each case on the total weight of the separated product:
- 14-methyl-16-oxabicyclo[10.3.1]pentadecene (1.1): 80 - 99% by weight,
- 3-methylcyclopentadecane-1,5-diol (II.1) : 0 - 5% by weight,
- 3-methylcyclopentadecane-1,5-dione (III.1): 0 - 5% by weight, preferably 0 to 1% by weight,
- 14-methyl-16-oxabicyclo[10.3.1]hexadecane (VI.1) : 0 - 15% by weight, preferably 0 to 10% by weight,
- solvent: 0 - 5% by weight, preferably 0 to 1% by weight,
- 3-methylcyclopentadecan-5-ol-1-one (VII.1): 0 - 5% by weight.

13. The method according to any of the preceding claims, wherein the solvent content of the reaction mixture in step b) is always maintained at at least 20% by weight, preferably at least 30% by weight, in particular at least 50% by weight, based on the total weight of the reaction mixture in the reaction zone.

14. A method for preparing conversion products of the compound of formula (I) as defined in any of the preceding claims, wherein in addition
d) the compounds of the general formula (I) are subjected to a reaction to obtain at least one compound of the general formula (IV) 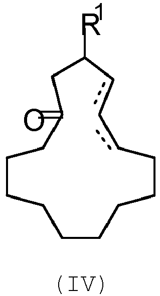 where the symbol ----- is in one case a single bond and in one case a double bond and R¹ is hydrogen or C₁-C₄-alkyl.

15. The method according to claim 14, wherein in addition
e) the compounds of the general formula (IV) are subjected to a hydrogenation to obtain the compound of the general formula (V) 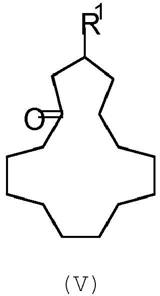 where R¹ is hydrogen or C₁-C₄-alkyl.

## Revendications

1. Procédé de fabrication de composés de la formule générale (I) 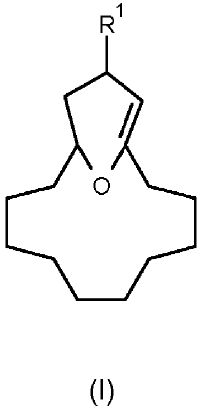 où R¹ représente hydrogène ou alkyle en C₁-C₄,
dans lequel
a) un matériau de départ est préparé, qui contient un composé de la formule générale II 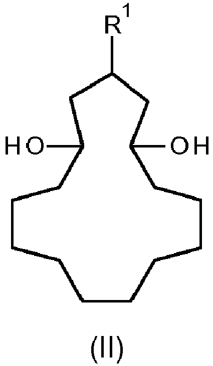
b) le matériau de départ préparé dans l'étape a) est soumis dans une zone de réaction à une réaction à une température dans une plage allant de 100 à 240 °C et une pression dans une plage allant de 0,1 à 150 mbar en présence d'un catalyseur hétérogène et d'un solvant ou d'un mélange de solvants, qui présente à 180 °C une pression de vapeur dans la plage allant de 10⁻⁵ à 100 mbar, et
c) le composé de la formule (I) est séparé par distillation à partir du mélange réactionnel.

2. Procédé selon la revendication 1, dans lequel R¹ représente hydrogène ou méthyle, notamment méthyle.

3. Procédé selon la revendication 1 ou 2, dans lequel la réaction dans l'étape b) comprend une première phase, pendant laquelle aucune fraction contenant le composé de la formule (I) n'est encore séparée par distillation à partir du mélange réactionnel.

4. Procédé selon l'une quelconque des revendications 1 à 3, dans lequel la pression de vapeur du solvant utilisé dans l'étape b) est inférieure à la pression de vapeur du diol (II).

5. Procédé selon l'une quelconque des revendications 1 à 3, dans lequel la pression de vapeur du solvant utilisé dans l'étape b) est comprise entre la pression de vapeur du composé (I) et la pression de vapeur du composé (II).

6. Procédé selon l'une quelconque des revendications 1 à 3, dans lequel la pression de vapeur du solvant utilisé dans l'étape b) est comprise entre la pression de vapeur du composé (I) et la pression de vapeur du composé (III) 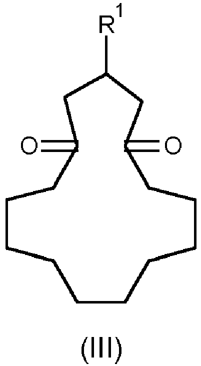 où R¹ représente hydrogène ou alkyle en C₁-C₄.

7. Procédé selon l'une quelconque des revendications précédentes, dans lequel le solvant utilisé dans l'étape b) est choisi parmi
- les hydrocarbures aliphatiques, cycloaliphatiques et aromatiques,
- les alcools aliphatiques, cycloaliphatiques et aromatiques mono- et polyvalents,
- les éther-alcools, les polyéther-polyols et leurs éthers mono- et dialkyliques, les éthers aromatiques et les éthers aliphatiques à chaîne ouverte,
- les cétones,
- les esters,
- les mélanges de ceux-ci.

8. Procédé selon l'une quelconque des revendications précédentes, dans lequel le solvant utilisé dans l'étape b) est choisi parmi
- les alcanes en C₁₀-C₃₀,
- les alcanols en C₆-C₃₀,
- les alcane-diols en C₂-C₃₀,
- les polyalkylène glycols et leurs éthers mono- et dialkyliques,
- les mélanges de ceux-ci.

9. Procédé selon l'une quelconque des revendications 1 à 8, dans lequel, dans l'étape c), le composé de la formule (1.1) est séparé à partir du mélange réactionnel par distillation en une étape, et le produit séparé contient les composés suivants, à chaque fois par rapport au poids total du produit séparé :
14-méthyl-16-oxabicyclo[10.3.1]pentadécène (I.1) : 75 à 95 % en poids,
3-méthyl-cyclopentadécane-1,5-diol (11.1) : 0 à 5 % en poids,
3-méthylcyclopentadécane-1,5-dione (III.1) : 1 à 10 % en poids,
14-méthyl-16-oxabicyclo[10.3.1]hexadécane (VI.1) : 0 à 15 % en poids.

10. Procédé selon l'une quelconque des revendications 1 à 8, dans lequel la séparation dans l'étape c) comprend une distillation fractionnée.

11. Procédé selon la revendication 10, dans lequel, dans l'étape c), pour la séparation par distillation d'une fraction contenant le composé de la formule (I), au moins une colonne de distillation est utilisée, qui comprend au moins 10 plateaux théoriques.

12. Procédé selon la revendication 10 ou 11 pour la fabrication de 14-méthyl-16-oxabicyclo[10.3.1]pentadécène (I.1), dans lequel, dans l'étape c), le composé de la formule (I) est séparé à partir du mélange réactionnel par distillation fractionnée, et le produit séparé contient les composés suivants, à chaque fois par rapport au poids total du produit séparé :
- 14-méthyl-16-oxabicyclo[10.3.1]pentadécène (I.1) : 80 à 99 % en poids,
- 3-méthyl-cyclopentadécane-1,5-diol (II.1) : 0 à 5 % en poids,
- 3-méthylcyclopentadécane-1,5-dione (III.1) : 0 à 5 % en poids, de préférence 0 à 1 % en poids,
- 14-méthyl-16-oxabicyclo[10.3.1]hexadécane (VI.1) : 0 à 15 % en poids, de préférence 0 à 10 % en poids,
- solvant : 0 à 5 % en poids, de préférence 0 à 1 % en poids,
- 3-méthyl-cyclopentadécan-5-ol-1-one (VII.1) : 0 à 5 % en poids.

13. Procédé selon l'une quelconque des revendications précédentes, dans lequel, dans l'étape b), la teneur du mélange réactionnel en le solvant est toujours maintenue à au moins 20 % en poids, de préférence au moins 30 % en poids, notamment au moins 50 % en poids, par rapport au poids total du mélange réactionnel dans la zone de réaction.

14. Procédé de fabrication de produits dérivés du composé de la formule (I) tel que défini dans l'une quelconque des revendications précédentes, dans lequel en outre
d) les composés de la formule générale (I) sont soumis à une réaction avec obtention d'au moins un composé de la formule générale (IV) 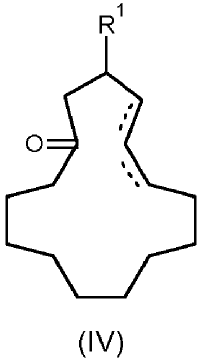 où le symbole représente une fois une simple liaison et une fois une double liaison, et R¹ représente hydrogène ou alkyle en C₁-C₄.

15. Procédé selon la revendication 14, dans lequel en outre
e) les composés de la formule générale (IV) sont soumis à une hydrogénation avec obtention du composé de la formule générale (V) 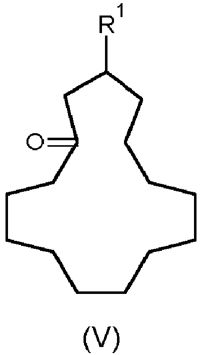 où R¹ représente hydrogène ou alkyle en C₁-C₄.
